# EUROPÄISCHE PATENTSCHRIFT

(11) **EP 1 991 643 B1**
(45) Veröffentlichungstag und Bekanntmachung des Hinweises auf die Patenterteilung: **30.10.2019**
(21) Anmeldenummer: 07726453.9
(22) Anmeldetag: 21.02.2007
(51) Int. Cl.: C10L 1/2383, C10L 1/233, C10L 1/238, C10L 1/222, C10M 159/16, C10L 10/18, C08G 73/02, C07D 265/16, C08G 61/12, C08G 14/073, C08F 8/32, C09K 15/24, C09K 15/30, C07C 215/50, C07C 217/58, C10M 133/48, C10M 149/12, C10M 159/12, C08K 5/3437

(54) **VERWENDUNG VON MEHRKERNIGEN PHENOLISCHEN VERBINDUNGEN ALS STABILISATOREN**
USE OF POLYNUCLEAR PHENOLIC COMPOUNDS AS STABILISERS
UTILISATION DE COMPOSÉS PHÉNOLIQUES À PLUSIEURS NOYAUX EN TANT QUE STABILISATEURS

(30) Priorität: 27.02.2006 EP 06003970
(43) Veröffentlichungstag der Anmeldung: 19.11.2008
(60) Teilanmeldung: 10180585.1
(73) Patentinhaber: BASF SE, 67056 Ludwigshafen am Rhein (DE)
(72) Erfinder: LANGE, Arno, 67098 Bad Dürkheim (DE); MACH, Helmut, 69115 Heidelberg (DE); RATH, Hans, Peter, 67269 Grünstadt (DE); POSSELT, Dietmar, 69120 Heidelberg (DE)
(74) Vertreter: BASF IP Association
(86) Internationale Anmeldenummer: PCT/EP2007/051632
(87) Internationale Veröffentlichungsnummer: WO 2007/099048

(56) Entgegenhaltungen:
- WO-A-01/25293
- WO-A1-01/25293
- WO-A1-01/25293
- DE-A1- 2 161 121
- DE-A1- 2 161 121
- US-A- 2 459 112
- US-A- 2 459 112
- US-A- 2 459 112
- GILLIAN R. GOWARD ET AL: "Benzoxazine Oligomers: Evidence for a Helical Structure from Solid-State NMR Spectroscopy and DFT-Based Dynamics and Chemical Shift Calculations", JOURNAL OF THE AMERICAN CHEMICAL SOCIETY, Bd. 125, Nr. 19, 1. Mai 2003 (2003-05-01), Seiten 5792-5800, XP055128804, ISSN: 0002-7863, DOI: 10.1021/ja029059r
- WOODGATE P D ET AL: "Synthesis of dioxazaborocines from N,N'-alkylbridged-bis(bis(2-hydroxybenzyl) aminomethyl)amines", JOURNAL OF ORGANOMETALLIC CHEMISTRY, ELSEVIER-SEQUOIA S.A. LAUSANNE, CH, Bd. 595, Nr. 2, 1. Februar 2000 (2000-02-01), Seiten 215-223, XP004187391, ISSN: 0022-328X, DOI: 10.1016/S0022-328X(99)00627-0
- VERNON M. RUSSELL ET AL: "Study of the characterization and curing of benzoxazines using13C solid-state nuclear magnetic resonance", JOURNAL OF APPLIED POLYMER SCIENCE, Bd. 70, Nr. 7, 14. November 1998 (1998-11-14), Seiten 1413-1425, XP055233795, US ISSN: 0021-8995, DOI: 10.1002/(SICI)1097-4628(19981114)70:7<1413 ::AID-APP16>3.0.CO;2-0
- GILLIAN R. GOWARD ET AL: "Benzoxazine Oligomers: Evidence for a Helical Structure from Solid-State NMR Spectroscopy and DFT-Based Dynamics and Chemical Shift Calculations", JOURNAL OF THE AMERICAN CHEMICAL SOCIETY, vol. 125, no. 19, 1 May 2003 (2003-05-01), pages 5792-5800, XP055128804, ISSN: 0002-7863, DOI: 10.1021/ja029059r
- WOODGATE P D ET AL: "Synthesis of dioxazaborocines from N,N'-alkylbridged-bis(bis(2-hydroxybenzyl) aminomethyl)amines", JOURNAL OF ORGANOMETALLIC CHEMISTRY, ELSEVIER-SEQUOIA S.A. LAUSANNE, CH, vol. 595, no. 2, 1 February 2000 (2000-02-01), pages 215-223, XP004187391, ISSN: 0022-328X, DOI: 10.1016/S0022-328X(99)00627-0
- VERNON M. RUSSELL ET AL: "Study of the characterization and curing of benzoxazines using13C solid-state nuclear magnetic resonance", JOURNAL OF APPLIED POLYMER SCIENCE, vol. 70, no. 7, 14 November 1998 (1998-11-14), pages 1413-1425, XP055233795, US ISSN: 0021-8995, DOI: 10.1002/(SICI)1097-4628(19981114)70:7<1413 ::AID-APP16>3.0.CO;2-0

## Beschreibung

Die vorliegende Erfindung betrifft die Verwendung von speziellen mehrkernigen phenolischen Verbindungen als Stabilisatoren zur Stabilisierung von unbelebtem organischen Material gegen die Einwirkung von Licht, Sauerstoff und Wärme, insbesondere in Turbinenkraftstoffen (jet fuels) und Schmierstoffzusammensetzungen. Weiterhin betrifft die vorliegende Erfindung eine Turbinenkraftstoffzusammensetzung, welche diese mehrkernigen phenolischen Verbindungen enthalten.

Die mechanischen, chemischen und/oder ästhetischen Eigenschaften von unbelebtem organischen Material, z. B. von Kunststoffen und Lacken, aber auch von Mineralölprodukten und Kraftstoffen, werden bekanntermaßen durch die Einwirkung von Licht, Sauerstoff und Wärme verschlechtert. Diese Verschlechterung zeigt sich üblicherweise als Vergilbung, Verfärbung, Rissbildung oder Versprödung des Materials. Es sind schon Stabilisatoren oder Stabilisatorzusammensetzungen bekannt, mit denen ein verbesserter Schutz gegen eine solche Beeinträchtigung von organischem Material durch Licht, Sauerstoff und Wärme erzielt werden kann.

So werden in der WO 05/073152 (1) 2-Alkyl-polyisobutenylphenole und deren Mannich-Addukte als Antioxidantien zur Stabilisierung von unbelebtem organischen Material gegen die Einwirkung von Licht, Sauerstoff und Wärme beschrieben. Als zu stabilisierende Materialien werden auch Kraftstoffe wie Ottokraftstoffe, Dieselkraftstoffe und Turbinenkraftstoffe sowie Schmierstoffzusammensetzungen genannt. In Turbinenkraftstoffen bewirken diese 2-Alkyl-polyisobutenyl-phenole und deren Mannich-Addukte eine Verbesserung der Thermostabilität sowie eine Verringerung der Ablagerungen im Kraftstoffkreislauf und Verbrennungssystem der Turbinen.

Auch die WO 03/106595 (2) offenbart neben hydrocarbylsubstituierten Bernsteinsäurederivaten und Polyalkenylthiophosphonatestern Mannich-Addukte aus hydrocarbylsubstituierten Phenolen, einem Aldehyd und einem Amin als Additive für Turbinenkraftstoffe (jet fuels) zur Verbesserung der Thermostabilität sowie zur Verringerung von Ablagerungen.

Tetrahydrobenzoxazine mit einem Benzolkern sind als Zusatzstoffe für Kraftstoff- und Schmierstoffzusammensetzungen bekannt. So offenbaren die WO 01/25293 (3) und die WO 01/25294 (4) derartige Tetrahydrobenzoxazine mit längerkettigen Resten wie Polyisobutenylresten, welche als Substituenten am Benzolkern sitzen, als ventilreinigende und ventilreinhaltende Ottokraftstoffdetergentien. Diese Tetrahydrobenzoxazine werden gemäß den in (3) und (4) genannten Herstellverfahren als Gemische mit den entsprechenden offenkettigen Mannich-Addukten des zugrundeliegenden Phenols erhalten und auch so in den Ottokraftstoffen eingesetzt.

Im Jounal of Polymer Science, Part A, Polymer Chemistry, Vol. 31, Seite 1941-1958 (5) beschreiben D. Jamois, M. Tessier und E. Marechal Herstellverfahren für Polyisobutenb-polyethylenamine, welche sich als Schlammdispergiermittel in Motorenölen eignen. Unter den von diesen Autoren hergestellten Verbindungen sind unter anderem auch zweikernige phenolische Verbindungen, die über die jeweiligen ortho-Positionen der beiden Phenolkerne durch eine -CH₂-N(C₁₂H₂₅)-CH₂-Brücke verknüpft sind.

Aus der US 6 323 270 B1 (6) sind Oligomere oder Polymere von Tetrahydrobenzoxazinen bekannt, welche am Stickstoffatom und/oder am Benzolkern Alkylgruppen mit 1 bis 10 Kohlenstoffatomen oder Arylgruppen, Alkylarylgruppen oder Arylalkylgruppen mit jeweils 6 bis 20 Kohlenstoffatomen als Substituenten tragen können. Die genaue chemische Struktur solcher Tetrahydrobenzoxazin-Oligomerer oder -polymerer wird nicht angegeben. Diese Tetrahydrobenzoxazin-Oligomere oder -polymere eignen sich zur Herstellung von Bremsbelägen für Flugzeuge, keramischen Artikeln, Verpackungsmaterialien, Beschichtungen, Klebstoffe und Kompositmaterialien.

In der JP 2003-255 487 A (7) werden polymere mehrkernige phenolische Verbindungen offenbart, welche über die ortho-Positionen der Phenolkerne jeweils durch -CH₂-N(C₁-C₂₅-Alkyl)-CH₂-Brücken oder -CH₂-N(Phenyl)-CH₂-Brücken verknüpft sind. Auch die Phenolkerne selbst können durch C₁-C₂₅-Alkylreste substituiert sein. Die Anzahl der Phenolkerne in den Polymermolekülen wird mit 2 bis 1002 angegeben. Als konkrete Beispiele für die auftretenden C₁-C₂₅-Alkylreste werden Methyl, Ethyl, Butyl, Octyl und Dodecyl genannt. Substituenten, die eine oligomere oder polymere Verteilung aufweisen, werden nicht genannt. Diese polymeren mehrkernigen phenolischen Verbindungen werden als photothermographisch bilderzeugendes Material empfohlen.

Aus der US 2,459,112 sind Mannich-Addukte aus langkettig alkylierten Phenolen und Polyaminen bekannt, welche in Kraftstoffzusammensetzungen (z.B. Turbinenkraftstoffen) Verwendung finden, u.a. um deren Oxidationstabilität zu verbessern.

Es besteht - insbesondere für den Mineralölprodukte- und Kraftstoff-Bereich - ein Bedarf an Stabilisatoren bzw. Antioxidantien mit verbesserter Schutzwirkung gegen die Beeinträchtigung der Materialeigenschaften durch Licht, Sauerstoff und Wärme. Vor allem für Turbinenkraftstoffe (jet fuels), die einer extremen thermischen Belastung beim und vor dem Verbrennungsvorgang in Turbinen, beispielsweise in Flugzeugturbinen, ausgesetzt sind, werden neue verbesserte Stabilisatoren gesucht. Diese sollen in den Turbinen gleichzeitig über ihre Wirkungsweise als Antioxidantien und/oder Dispergatoren auch Ablagerungen im Kraftstoffkreislauf und im Verbrennungssystem verringern. Weiterhin werden neue verbesserte Stabilisatoren für Schmierstoffzusammensetzungen gesucht, welche insbesondere einen verbesserten Schutz gegen das Oxidations- und Alterungsverhalten und/oder eine verbesserte Scherstabilität bieten.

Es bestand daher die Aufgabe, Stabilisatoren mit einer verbesserten Stabilisierung von unbelebtem organischen Material, insbesondere von Mineralölprodukten und Kraftstoffen, vor allem von Turbinenkraftstoff und von Schmierstoffzusammensetzungen, gegen die Einwirkung von Licht, Sauerstoff und Wärme bereitzustellen.

Demgemäß betrifft die Erfindung die Verwendung von phenolischen Verbindungen gemäß Anspruch 1. Es wurde die Verwendung von mehrkernigen phenolischen Verbindungen mit bis zu 20 Benzolkernen pro Molekül, welche durch Umsetzung eines Tetrahydrobenzoxazins der allgemeinen Formel I 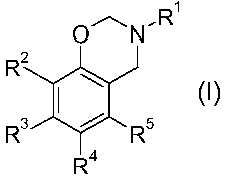 in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet,
wobei R⁶ ein Wasserstoffatom oder einen C₁- bis C₄-Alkylrest bezeichnet, und
in der die Substituenten R², R³, R⁴ und R⁵ unabhängig voneinander für Wasserstoffatome, Hydroxylgruppen oder Hydrocarbylreste mit jeweils 1 bis 3000 Kohlenstoffatomen, stehen, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
mit einem oder mehreren der gleichen oder verschiedener Phenole der allgemeinen Formel II 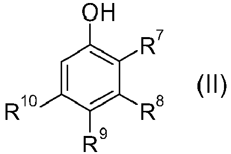 in der die Substituenten R⁷, R⁸, R⁹ und R¹⁰ unabhängig voneinander für Wasserstoffatome, Hydroxylgruppen oder Hydrocarbylreste mit jeweils 1 bis 3000 Kohlenstoffatomen, stehen, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
und/oder mit einem oder mehreren der gleichen oder verschiedener Tetrahydrobenzoxazine der allgemeinen Formel I,
wobei der Substituent R⁴ auch für einen Rest der Formel Z und der Substituent R⁹ auch für einen Rest der Formel Z' stehen kann 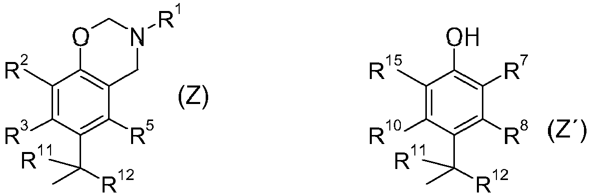 in denen die Substituenten R¹, R², R³, R5, R⁷, R⁸ und R¹⁰ die vorgenannten Bedeutungen haben, der Substituent R⁷ auch ein aus einem Tetrahydrobenzoxazin der allgemeinen Formel I abgeleiteter Rest sein kann, der Sustituent R¹⁵ für Wasserstoff oder einen aus einem Tetrahydrobenzoxazin der allgemeinen Formel I abgeleiteten Rest steht und die Substituenten R¹¹ und R¹² gleich oder verschieden sein können und Wasserstoff oder einen C₁- bis C₁₀-Alkylrest bezeichnen,
und in der die Substituenten R² und R³ oder R³ und R⁴ oder R⁴ und R⁵ mit der an den Benzolkern angebundenen Teilstruktur -O-CH₂-NR¹³-CH₂- auch einen zweiten Tetrahydrooxazin-Ring oder die Substituenten R² und R³ und R⁴ und R⁵ mit den an den Benzolkern angebundenen Teilstrukturen -O-CH₂-NR¹³-CH₂- und -O-CH₂-NR¹⁴-CH₂- auch einen zweiten und einen dritten Tetrahydrooxazin-Ring ausbilden können, wobei R¹³ und R¹⁴ unabhängig voneinander Hydrocarbylreste mit jeweils 1 bis 3000 Kohlenstoffatomen bedeutet, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
mit der Maßgabe, dass mindestens einer der Substituenten R¹, R², R³, R⁴, R⁵, R⁷, R⁸, R⁹, R¹⁰, R¹³ oder R¹⁴ 13 bis 3000 Kohlenstoffatome aufweist und die übrigen Substituenten aus der Gruppe R¹, R², R³, R⁴, R⁵, R⁷, R⁸, R⁹, R¹⁰, R¹³ oder R¹⁴, wenn sie für Hydrocarbylreste stehen, jeweils 1 bis 20 Kohlenstoffatome aufweisen,
erhältlich sind, als Stabilisatoren zur Stabilisierung von unbelebtem organischen Material gegen die Einwirkung von Licht, Sauerstoff und Wärme gefunden.

Die strukturelle Besonderheit der zu verwendenden mehrkernigen phenolischen Verbindungen ist, dass sie mindestens einen längerkettigen Hydrocarbylrest mit 13 bis 3000 Kohlenstoffatomen als einen der Substituenten R¹, R², R³, R⁴, R⁵, R⁷, R⁸, R⁹, R¹⁰, R¹³ oder R¹⁴, welche aus den eingesetzten Tetrahydrobenzoxazinen I oder den Phenolen II stammen, enthalten. In einer bevorzugten Ausführungsform ist dieser längerkettige Hydrocarbylrest mit 13 bis 3000 Kohlenstoffatomen ein Polyisobutenylrest. Der genannte längerkettige Hydrocarbylrest kann in einer weiteren Ausführungsform auch einen C₁₆- bis C₂₀-Alkyl- oder -Alkenylrest bedeuten. Insbesondere sitzt dieser längerkettige Hydrocarbylrest, der vorzugsweise ein Polyisobutenylrest ist, an einem Oxazinring oder an einem Benzolkern in ortho- oder vorzugsweise in paraStellung zur phenolischen Hydroxylgruppe, d.h. er tritt als Substituent R¹ oder R² oder R⁴ oder R⁷ oder R⁹ oder R¹³ oder R¹⁴ auf. Dieser längerkettige Hydrocarbylrest, der vorzugsweise ein Polyisobutenylrest ist, umfasst vorzugsweise 21 bis 3000 oder vorzugsweise 21 bis 1000, insbesondere 26 bis 3000 oder insbesondere 26 bis 500, vor allem 30 bis 3000 oder vor allem 30 bis 250 Kohlenstoffatome. Im Falle von Polyisobutenylresten weisen diese zahlenmittlere Molekulargewichte Mₙ von 183 bis 42.000, vorzugsweise 500 bis 15.000, insbesondere 700 bis 7000, vor allem 900 bis 3000, ganz besonders bevorzugt 900 bis 1100 auf.

Als C₁₆- bis C₂₀-Alkyl- oder -Alkenylreste eignen sich zweckmäßigerweise die Reste von entsprechenden gesättigten oder ungesättigten Fettalkoholen mit 16 bis 20 Kohlenstoffatomen. Insbesondere sind hier n-Hexadecyl (Palmityl), n-Octadecyl (Stearyl), n-Eicosyl, Oleyl, Linolyl und Linolenyl zu nennen, welche gemäß ihrem natürlichen Vorkommen meist als technische Gemische untereinander auftreten.

Der genannte längerkettige Hydrocarbylrest mit 13 bis 3000 Kohlenstoffatomen kann in den mehrkernigen phenolischen Verbindungen auch mehrfach, beispielsweise zweifach oder dreifach, vertreten sein. In einer bevorzugten Ausführungsform treten ein oder zwei Polyisobutenyl-Reste mit einem jeweiligen zahlenmittleren Molekulargewicht Mₙ von 183 bis 42.000 im Molekül als Substituent R¹ und/oder R² und/oder R⁴ und/oder R⁷ und/oder R⁹ und/oder R¹³ und/oder R¹⁴ auf.

Die übrigen Substituenten aus der Gruppe R¹, R², R³, R⁴, R⁵, R⁷, R⁸, R⁹, R¹⁰, R¹³ oder R¹⁴, die nicht für Substituenten mit 13 bis 3000 Kohlenstoffatomen bzw. für Polyisobutenylreste mit einem zahlenmittleren Molekulargewichte Mₙ von 183 bis 42.000 stehen, bezeichnen unabhängig voneinander Wasserstoffatome, Hydroxylgruppen oder, wenn sie für Hydrocarbylreste stehen, meist kürzerkettige Hydrocarbylreste mit 1 bis 20, vorzugsweise 1 bis 12, vor allem 1 bis 8, ganz besonders bevorzugt lineare oder verzweigte C₁- bis C₄-Alkylreste. Typische Beispiele für die letzteren sind Methyl, Ethyl, n-Propyl, iso-Propyl, n-Butyl, 2-Butyl, sec.-Butyl und tert.-Butyl. Methylreste und tert.-Butylreste werden hierbei ganz besonders bevorzugt.

Bevorzugt einzusetzende mehrkernige phenolische Verbindungen Tetrahydrobenzoxazine I sind auch solche, bei denen die aus den eingesetzten Tetrahydrobenzoxazinen I bzw. Phenolen II stammenden Substituenten R² und/oder R⁴ und/oder R⁷ und/oder R⁹, wenn sie für kürzerkettige Hydrocarbylreste stehen, lineare oder verzweigte C₁- bis C₄-Alkylreste, insbesondere Methylreste und/oder tert.-Butylreste, bezeichnen. Derartige Substitutionsmuster kommen natürlich nur bei Tetrahydrobenzoxazine I mit insgesamt einem oder zwei Tetrahydrooxazin-Ringsystemen in Betracht.

Im Rest der Formel Z bzw. Z' bezeichnen die Substituenten R¹¹ und R¹² vorzugsweise Wasserstoff und/oder lineare oder verzweigte C₁- bis C₄-Alkylreste, insbesondere Methylreste. Die Verbindungen I und II mit einem Rest Z bzw. Z', bei dem R¹¹ = R¹² = Methyl ist, leiten sich von Bisphenol A [2,2-Bis-(4-hydroxyphenyl)propan] ab. Herstellungsbedingt können Verbindungen I mit einem Rest Z und Verbindungen I mit dem entsprechenden Rest Z' auch als Mischungen vorliegen.

Unter Hydrocarbylresten mit 1 bis 3000 bzw. 13 bis 3000 Kohlenstoffatomen für die Substituenten R¹, R², R³, R⁴, R⁵, R⁷, R⁸, R⁹, R¹⁰, R¹³ und R¹⁴ sollen hier reine Kohlenwasserstoffreste jeglicher Struktur verstanden werden, die definitionsgemäß auch noch durch ein oder mehrere Heteroatome aus der Gruppe O und S und/oder durch eine oder mehrere Gruppierungen NR⁶ unterbrochen sein können. Ein typischer durch eine NR⁶-Gruppierung unterbrochener Hydrocarbylrest leitet sich von 3-(Dimethylamino)-propylamin ab. Insbesondere sind Hydrocarbylreste Alkyl-, Alkenyl-, Cycloalkyl-, Aryl-, Alkylaryl-, Alkenylaryl- oder Arylalkyl-Reste.

Bei Unterbrechungen des Hydrocarbylrestes durch Gruppierungen NR⁶ sind auch solche Reste gemeint, bei denen am Ende die Gruppierungen NR⁶ formal in eine C-H-Bindung insertiert ist, also beispielsweise Substituenten R¹, R², R³, R⁴, R⁵, R⁷, R⁸, R⁹, R¹⁰, R¹³ oder R¹⁴ mit einer NH₂-Endgruppe. Derartige Hydrocarbylreste leiten sich z.B. von Polyaminen wie Ethylendiamin, Diethylentriamin, Triethylentetramin, Tetraethylenpentamin, etc. ab, bei denen eines der endständigen Stickstoffatome das N-Atom im Oxazin-Ring darstellt.

Der Ausdruck "Alkyl" umfasst geradkettige und verzweigte Alkylgruppen. Beispiele für Alkylgruppen sind neben den bereits oben genannten Methyl-, Ethyl-, n-Propyl-, iso-Propyl-, n-Butyl-, 2-Butyl-, sec.-Butyl- und tert.-Butyl-Resten insbesondere auch n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethyl-butyl, 2,2-Dimethylbutyl, 3,3-Dimethylbutyl, 1,1,2-Trimethylpropyl, 1,2,2-Trimethyl-propyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, n-Nonyl, n-Decyl, n-Dodecyl, n-Tridecyl, iso-Tridecyl, n-Tetradecyl (Myristyl), n-Hexadecyl (Palmityl), n-Octadecyl (Stearyl), und n-Eicosyl.

Beispiele für Alkenylreste sind Vinyl, 1-Propenyl, 2-Propenyl, Oleyl, Linolyl und Linolenyl.

Beispiele für Cylcoalkylreste sind C₅- bis C₇-Cycloalkylgruppen wie Cyclopentyl, Cyclohexyl und Cycloheptyl, welche noch durch Alkylgruppen, beispielsweise Methylreste, substituiert sein können.

Der Ausdruck "Aryl" umfasst einkernige, zweikernige, dreikernige und höherkernige aromatische Kohlenwasserstoffreste. Diese Arylreste können im Falle einer Substitution durch die beispielsweise vorgenannten Alkyl- und/oder Alkenylreste zu Alkylaryl- bzw. Alkenylarylresten noch 1, 2, 3, 4 oder 5, vorzugsweise 1, 2 oder 3 Substituenten tragen. Typische Beispiele sind Phenyl, Tolyl, Xylyl, Mesityl, Naphthyl, Fluorenyl, Anthracenyl, Phenanthrenyl, Naphthacenyl und Styryl. Ein typisches Beispiel für einen Arylalkylrest ist Benzyl.

Ist der längerkettige Hydrocarbylrest mit 13 bis 3000 Kohlenstoffatomen ein Polyisobutenylrest, kann er auf prinzipiell jedem gängigen und kommerziell erhältlichen Polyisobuten basieren, welches in geeigneter Weise in die Synthese der Tetrahydrobenzoxazine I eingebracht wird. Ein solches Polyisobuten besitzt ein zahlenmittleres Molekulargewicht Mₙ von mindestens 183. Bevorzugt sind Polyisobutene mit einem zahlenmittleren Molekulargewicht Mₙ im Bereich von 183 bis 42.000, besonders bevorzugt von 500 bis 15.000, vor allem von 700 bis 7000, insbesondere von 900 bis 3000 und ganz besonders bevorzugt von 900 bis 1100. Unter den Begriff "Polyisobuten" fallen auch oligomere Isobutene wie dimeres, trimeres, tetrameres, pentameres, hexameres und heptameres Isobuten.

Vorzugsweise leiten sich die in den verwendeten mehrkernigen phenolischen Verbindungen eingebauten Polyisobutenylreste von sogenanntem "reaktiven" Polyisobuten ab. Reaktive Polyisobutene unterscheiden sich von den konventionellen Polyisobutenen durch den Gehalt an terminal angeordneten Doppelbindungen, d.h. an Vinyliden-Doppelbindungen [-CH-C(CH₃)=CH₂] (α-Olefin) oder Vinyl-Doppelbindungen [-CH=C(CH₃)₂] (β-Olefin). So enthalten reaktive Polyisobutene wenigstens 50 Mol-% terminal angeordnete Doppelbindungen, bezogen auf die Gesamtanzahl an Polyisobuten-Makromolekülen. Besonders bevorzugt sind Polyisobutene mit wenigstens 60 Mol-% und insbesondere mit wenigstens 80 Mol-% terminal angeordnete Doppelbindungen, bezogen auf die Gesamtanzahl an Polyisobuten-Makromolekülen. Außerdem weisen die im Wesentlichen homopolymere Polyisobutenylreste einheitliche Polymergerüste auf. Darunter werden solche Polyisobuten-Systeme verstanden, die zu wenigstens 85 Gew.-%, vorzugsweise zu wenigstens 90 Gew.-%, besonders bevorzugt zu wenigstens 95 Gew.-% und insbesondere zu wengistens 99 Gew.-% aus Isobuteneinheiten der Wiederholungseinheit [-CH₂C(CH₃)₂-] aufgebaut sind.

Ein weiteres bevorzugtes Merkmal der Polyisobutene, die den verwendeten mehrkernigen phenolischen Verbindungen zugrunde liegen können, ist, dass sie zu wenigstens 15 Gew.-%, insbesondere zu wenigstens 50 Gew.-%, vor allem zu wenigstens 80 Gew.-% mit einer tert.-Butylgruppe [-CH₂C(CH₃)₃] terminiert sind.

Weiterhin weisen die als Basis für die als Ausgangsmaterial für die verwendeten mehrkernigen phenolischen Verbindungen verwendeten Tetrahydrobenzoxazine I bzw. Phenole II vorzugsweise dienenden Polyisobutene vorzugsweise einen Polydispersitätsindex (PDI) von 1,05 bis 10, vorzugsweise von 1,05 bis 3,0, insbesondere von 1,05 bis 2,0 auf. Unter Polydispersität versteht man den Quotienten aus gewichtsmittlerem Molekulargewicht M_{w} und zahlenmittlerem Molekulargewicht Mₙ (PDI = M_{w}/Mₙ). In einer bevorzugten Ausführungsform beträgt der gemittelte Polydispersitätsindex PDI für die Polyisobutenyl-Reste im den mehrkernigen phenolischen Verbindungen höchstens das 5-fache, vorzugsweise höchstens das 3-fache, insbesondere höchstens das 2-fache, vor allem höchstens das 1,5-fache, des gemittelten Polydispersitätsindex PDI für die Polyisobutenyl-Reste in den zugrundeliegenden Tetrahydrobenzoxazinen I und/oder Phenolen II.

Unter als Basis für die Tetrahydrobenzoxazine I bzw. Phenole II vorzugsweise dienenden Polyisobutenen werden auch alle durch kationische Polymerisation erhältlichen Polymerisate verstanden, die vorzugsweise wenigstens 60 Gew.-% Isobuten, besonders bevorzugt wenigstens 80 Gew.-%, vor allem wenigstens 90 Gew.-% und insbesondere wenigstens 95 Gew.-% Isobuten einpolymerisiert enthalten. Daneben können die Polyisobutene weitere Buten-Isomere wie 1- oder 2-Buten sowie davon verschiedene olefinisch ungesättigte Monomere, die mit Isobuten unter kationischen Polymerisationsbedingungen copolymerisierbar sind, einpolymerisiert enthalten.

Als Isobuten-Einsatzstoffe für die Herstellung von Polyisobutenen, die als Basis für die Tetrahydrobenzoxazine I bzw. Phenole II dienen können, eignen sich dementsprechend sowohl Isobuten selbst als auch isobutenhaltige C₄-Kohlenwasserstoffströme, beispielsweise C₄-Raffinate, C₄-Schnitte aus der Isobuten-Dehydrierung, C₄-Schnitte aus Steamcrackern, FCC-Crackern (FCC: Fluid Catalyzed Cracking), sofern sie weitgehend von darin enthaltenem 1,3-Butadien befreit sind. Besonders geeignete C₄-Kohlenwasserstoffströme enthalten in der Regel weniger als 500 ppm, vorzugsweise weniger als 200 ppm Butadien. Bei Einsatz von C₄-Schnitten als Einsatzmaterial übernehmen die von Isobuten verschiedenen Kohlenwasserstoffe die Rolle eines inerten Lösungsmittels.

Als mit Isobuten copolymerisierbare Monomere kommen Vinylaromaten wie Styrol und a-Methylstyrol, C₁-C₄-Alkylstyrole wie 2-, 3- und 4-Methylstyrol, sowie 4-tert.-Butylstyrol, Isoolefine mit 5 bis 10 C-Atomen wie 2-Methylbuten-1, 2-Methylpenten-1, 2-Methylhexen-1, 2-Ethylpenten-1, 2-Ethylhexen-1 und 2-Propylhepten-1 in Betracht.

Typische Polyisobutene, die als Basis für die Tetrahydrobenzoxazine I bzw. Phenole II dienen können, sind beispielsweise die Glissopal®-Marken der BASF Aktiengesellschaft, z. B. Glissopal 550, Glissopal 1000 und Glissopal 2300, sowie die Oppanol®-Marken der BASF Aktiengesellschaft, z.B. Oppanol B10, B12 und B15.

Neben Polyisobutenylresten können als längerkettige Hydrocarbylreste auch solche auftreten, die sich von Oligomeren oder Polymeren von C₂- bis C₁₂-Olefinen ableiten und im Mittel 13 bis 3000 Kohlenstoffatome aufweisen. Derartige meist polydisperse Hydrocarbylreste mit polymerer Verteilung sind beispielsweise solche, die sich von Ethylen, Propylen, Buten, Styrol, Methylstyrol, Hexen-1, Octen-1, Decen-1 oder Dodecen-1 ableiten. Sie können Homo- oder Copolymer-Reste sein. Ihr zahlenmittleres Molekulargewicht Mₙ beträgt mindestens 183, ihr Polydispersitätsindex PDI üblicherweise 1,05 bis 10. Bei niedermolekularen Resten mit Mₙ von 183 bis ca. 500 können sie auch monodispers vorliegen.

In einer bevorzugten Ausführungsform weisen die verwendeten mehrkernigen phenolischen Verbindungen ein mittleres Molekulargewicht Mₙ von 411 bis 25.000 auf. So steht beispielsweise das Molekulargewicht Mₙ von 411 für die kleinsten Vertreter der mehrkernigen phenolischen Verbindungen im Rahmen der vorliegenden Erfindung, nämlich Bis-(ortho- oder para-hydroxybenzyl)-tridecylamin. Besonders bevorzugte Bereiche für Mₙ sind 523 bis 25.000 oder 523 bis 17.000, insbesondere 593 bis 25.000 oder 593 bis 10.000, vor allem 649 bis 25.000 oder 649 bis 5000.

Beispiele für typische mehrkernige phenolische Verbindungen sind die folgenden, wobei "PIB" einen von einem hochreaktiven Polyisobuten (Mₙ 1000) abgeleiteten Polyisobutenylrest bezeichnet: 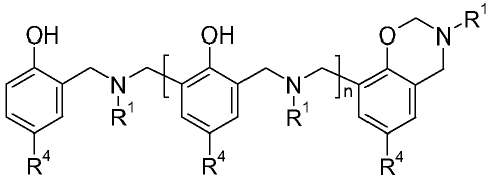
(IIIa) n = 0, R¹ = PIB, R⁴ = H
(IIIb) n = 0, R¹ = Methyl, R⁴ = PIB
(IIIc) n = 0, R¹ = PIB, R⁴ = tert.-Butyl
(IIId) n = 1, R¹ = PIB, R⁴ = H
(IIIe) n = 1, R¹ = Methyl, R⁴ = PIB
(IIIf) n = 1, R¹ = PIB, R⁴ = tert.-Butyl
(IIIg) n = 2, R¹ = PIB, R⁴ = H
(IIIh) n = 2, R¹ = Methyl, R⁴ = PIB
(IIIi) n = 2, R¹ = PIB, R⁴ = tert.-Butyl
(IIIj) n = 3, R¹ = PIB, R⁴ = H
(IIIk) n = 3, R¹ = Methyl, R⁴ = PIB
(IIIl) n = 3, R¹ = PIB, R⁴ = tert.-Butyl
(IIIm) n = 4, R¹ = PIB, R⁴ = H
(IIIn) n = 4, R¹ = Methyl, R⁴ = PIB
(IIIo) n = 4, R¹ = PIB, R⁴ = tert.-Butyl
(IIIp) n = 5, R¹ = PIB, R⁴ = H
(IIIq) n = 5, R¹ = Methyl, R⁴ = PIB
(IIIr) n = 5, R¹ = PIB, R⁴ = tert.-Butyl
(IIIs) n = 6, R¹ = PIB, R⁴ = H
(IIIt) n = 6, R¹ = Methyl, R⁴ = PIB
(IIIu) n = 6, R¹ = PIB, R⁴ = tert.-Butyl
(IIIv) n = 1, R¹ = Methyl,
   1 Rest R⁴ = PIB, 2 Reste R⁴ = tert.Butyl
(IIIw) n = 8, R¹ = Methyl,
   1 Rest R⁴ = PIB, 9 Reste R⁴ = tert.Butyl

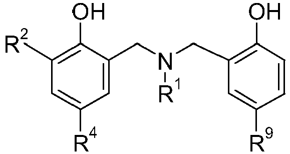

(IVa) R¹ = Methyl, R² = H, R⁴ = tert.-Butyl, R⁹ = PIB
(IVb) R¹ = Methyl, R² = R⁴ = tert.-Butyl, R⁹ = PIB
(IVc) R¹ = PIB, R² = R⁴ = tert.-Butyl, R⁹ = H
(IVd) R¹ = PIB, R² = R⁴ = R⁹ = H
(IVe) R¹ = PIB, R² = R⁴ = H, R⁹ = tert.-Butyl
(IVf) R¹ = PIB, R² = H, R⁴ = tert.-Butyl, R⁹ = PIB
(IVg) R¹ = PIB, R² = R⁴ = tert.-Butyl, R⁹ = PIB
(IVh) R¹ = PIB, R² = R⁴ = H,

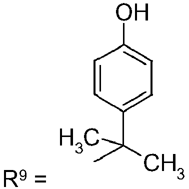

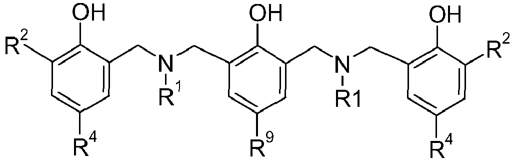

(Va) R¹ = Methyl, R² = R⁴ = H, R⁹ = PIB
(Vb) R¹ = Methyl, R² = R⁴ = tert.-Butyl, R⁹ = PIB
(Vc) R¹ = Methyl, R² = tert.-Butyl, R⁴ = Methyl, R⁹ = PIB
(Vd) R¹ = R² = Methyl, R⁴ = tert.-Butyl, R⁹ = PIB
(Ve) R¹ = 3-(Dimethylamino)propyl, R² = R⁴ = tert.-Butyl, R⁹ = PIB
(Vf) R¹ = PIB, R² = R⁴ = R⁹ = H
(Vg) R¹ = PIB, R² = R⁴ = H, R⁹ = tert.-Butyl
(Vh) R¹ = PIB, R² = R⁴ = tert.-Butyl, R⁹ = H
(Vi) R¹ = PIB, R² = H, R⁴ = R⁹ = tert.-Butyl
(Vj) R¹ = PIB, R² = R⁴ = R⁹ = tert.-Butyl
(Vk) R¹ = PIB, R² = R⁴ = H, R⁹ = PIB
(Vm) R¹ = PIB, R² = R⁴ = H, 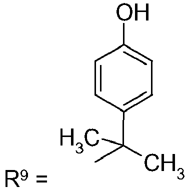
(Vn) R¹ = 3-(Dimethylamino)propyl, R² = tert.-Butyl, R⁴ = Methyl,
   R⁹ = PIB

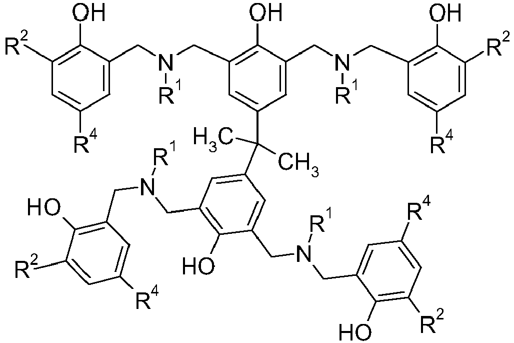

(VIa) R¹ = Methyl, R² = tert.-Butyl, 3 Reste R⁴ = tert.-Butyl, 1 Rest R⁴ = PIB
(VIb) R¹ = Methyl, R² = tert.-Butyl, 3 Reste R⁴ = Methyl, 1 Rest R⁴ = PIB
(VIc) R¹ = Methyl, 3 Reste R² = tert.-Butyl, 1 Rest R² = H,
   3 Reste R⁴ = tert.-Butyl, 1 Rest R⁴ (am Benzolkern mit R² = H) = PIB

Die beschriebenen mehrkernigen phenolischen Verbindungen der allgemeinen Formeln V und VI werden gemäß der vorliegenden Erfindung als Stabilisatoren zur Stabilisierung von unbelebtem organischen Material gegen die Einwirkung von Licht, Sauerstoff und Wärme verwendet. Hierunter ist insbesondere ihre Wirkungsweise als Antioxidantien im herkömmlichen Sinne zu verstehen. Dazu werden diese Verbindungen in das zu stabilisierende Material während oder nach dessen Herstellung eingearbeitet und möglichst homogen verteilt. Die Konzentration dieser Verbindungen in dem zu stabilisierenden organischen Material beträgt in der Regel 0,0001 bis 5 Gew.-%, vorzugsweise 0,001 bis 5 Gew.-%, vor allem 0,01 bis 2 Gew.-%, insbesondere 0,05 bis 1 Gew.-%, bezogen auf das organische Material.

Unter unbelebtem organischen Material sind beispielsweise kosmetische Präparate wie Salben und Lotionen, Arzneimittelformulierungen wie Pillen und Zäpfchen, photographische Aufzeichnungsmaterialien, insbesondere photographische Emulsionen, Anstrichmittel und Kunststoffe zu verstehen. Dazu zählen weiterhin und insbesondere Mineralölprodukte und Kraftstoffe, z.B. Dieselkraftstoff, Ottokraftstoff, Turbinenkraftstoff, Motorenöle, Schmieröle, Getriebeöle und Schmierfette.

Als Kunststoffe, die durch die beschriebenen mehrkernigen phenolischen Verbindungen stabilisiert werden können, seien beispielsweise genannt:
Polymere von Mono- oder Diolefinen, wie Polyethylen niedriger oder hoher Dichte, Polypropylen, lineares Polybuten-1, Polyisopren, Polybutadien sowie Copolymerisate von Mono- oder Diolefinen oder Mischungen der genannten Polymeren;
Polystyrol sowie Copolymere von Styrol oder a-Methylstyrol mit Dienen und/oder Acrylderivaten, z.B. Styrol-Butadien, Styrol-Acrylnitril (SAN), Styrol-Ethylmethacrylat, Styrol-Butadien-Ethylacrylat, Styrol-Acrylnitril-Methacrylat, Acrylnitril-Butadien-Styrol (ABS) oder Methylmethacrylat-Butadien-Styrol (MBS); halogenhaltige Polymere, z.B. Polyvinylchlorid, Polyvinylfluorid, Polyvinylidenfluorid sowie deren Copolymere;
Polymere, die sich von α,β -ungesättigten Säuren und deren Derivaten ableiten, wie Polyacrylate, Polymethacrylate, Polyacrylamide und Polyacrylnitrile;
Polymere, die sich von ungesättigten Alkoholen und Aminen bzw. von deren Acylderivaten oder Acetalen ableiten, z.B. Polyvinylalkohol und Polyvinylacetat;
Polyurethane, insbesondere thermoplastische Polyurethane, Polyamide, Polyharnstoffe, Polyphenylenether, Polyester, Polycarbonate, Polysulfone, Polyethersulfone und Polyetherketone.

Zu den Anstrichmitteln, die mit den beschriebenen mehrkernigen phenolischen Verbindungen stabilisiert werden können, zählen unter anderem Lacke wie Alkydharzlacke, Dispersionslacke, Epoxydharzlacke, Polyurethanlacke, Acrylharzlacke und Cellulosenitratlacke, oder Lasuren wie Holzschutzlasuren.

Die beschriebenen mehrkernigen phenolischen Verbindungen eignen sich in besonders vorteilhafter Weise als Stabilisatoren in Turbinenkraftstoffen (jet fuels). Hierunter ist auch ihre Wirkungsweise als Antioxidantien im herkömmlichen Sinne zu verstehen. Insbesondere dienen sie über ihre Wirkungsweise als Stabilisatoren zur Verbesserung der thermischen Stabilität von Turbinenkraftstoffen. Weiterhin verhindern sie insbesondere auch über ihre Wirkungsweise als Stabilisatoren, d.h. in ihrer Eigenschaft als Dispergatoren, Ablagerungen im Kraftstoffsystem und/oder Verbrennungssystem von Turbinen. Turbinenkraftstoff werden vor allem zum Betreiben von Flugzeugturbinen eingesetzt.

Ein weiterer Gegenstand der vorliegenden Erfindung ist eine Turbinenkraftstoffzusammensetzung, die einen Turbinenkraftstoff (jet fuel) und wenigstens eine der beschriebenen mehrkernigen phenolischen Verbindungen enthält.

Die erfindungsgemäße Turbinenkraftstoffzusammensetzung enthält eine Hauptmenge eines flüssigen Turbinenkraftstoffs, wobei es sich beispielsweise um einen in der zivilen oder militärischen Luftfahrt üblichen Turbinenkraftstoff handelt. Dazu zählen beispielsweise Kraftstoffe der Bezeichnung Jet Fuel A, Jet Fuel A-1, Jet Fuel B, Jet Fuel JP-4, JP-5, JP-7, JP-8 und JP-8+100. Jet A und Jet A-1 sind kommerziell erhältliche Turbinenkraftstoffspezifikationen auf Kerosinbasis. Die zugehörigen Normen sind ASTM D 1655 sowie DEF STAN 91-91. Jet B ist ein weiter geschnittener Kraftstoff auf Basis von Naphtha- und Kerosinfraktionen. JP-4 ist äquivalent zu Jet B. JP-5, JP-7, JP-8 und JP-8+100 sind militärische Turbinenkraftstoffe, wie sie beispielsweise von der Marine und Luftwaffe eingesetzt werden. Zum Teil bezeichnen diese Normen Formulierungen, die bereits weitere Additive, wie Korrosionsinhibitoren, Vereisungsinhibitoren, statische Dissipatoren, etc. enthalten.

Die beschriebenen mehrkernigen phenolischen Verbindungen können dem Turbinenkraftstoff oder der Turbinenkraftstoffzusammensetzung einzeln, als Gemische und gegebenenfalls in Kombination mit weiteren an sich bekannten Zusatzstoffen zugegeben werden.

Geeignete Zusatzstoffe, die in der erfindungsgemäßen Turbinenkraftstoffzusammensetzung enthalten sein können, umfassen üblicherweise Detergenzien, Korrosionsinhibitoren, weitere Antioxidantien wie sterisch gehinderte tert.-Butylphenole, N-Butylphenylendiamine oder N,N'-Diphenylamin und Derivate hiervon, Metalldesaktivatoren wie N,N'-Disalicyliden-1,2-diaminopropan, Lösungsvermittler, Antistatika wie Stadis 450, Biocide, Anti-Icing-Mittel wie Diethylenglykolmethylether, sowie Mischungen der genannten Zusatzstoffe.

Im Rahmen der vorliegenden Erfindung bevorzugte Zusatzstoffe sind die nachfolgend aufgeführten speziellen Verbindungsklassen (A), (B) und (C):
Bevorzugte Zusatzstoffe (A) sind von Bernsteinsäureanhydrid abgeleitete Verbindungen mit langkettigen Kohlenwasserstoffresten mit in der Regel 15 bis 700, vor allem 30 bis 200 Kohlenstoffatomen. Diese Verbindungen können weitere funktionelle Gruppen aufweisen, die vorzugsweise ausgewählt sind unter Hydroxy-, Amino-, Amido- und/oder Imidogruppen. Bevorzugte Additive sind die entsprechenden Derivate von Polyalkenylbernsteinsäureanhydrid, welche z. B. durch Umsetzung von Polyalkenen mit Maleinsäureanhydrid auf thermischem Weg oder über die chlorierten Kohlenwasserstoffe erhältlich sind. Das zahlenmittlere Molekulargewicht der langkettigen Kohlenwasserstoffreste liegt vorzugsweise in einem Bereich von etwa 200 bis 10.000, besonders bevorzugt 400 bis 5000, insbesondere 600 bis 3000 und speziell 650 bis 2000. Vorzugsweise leiten sich diese langkettigen Kohlenwasserstoffreste von konventionellen und insbesondere von den zuvor genannten reaktiven Polyisobutenen ab. Von besonderem Interesse als Zusatzstoffe (A) sind die Derivate von Polyalkenylbernsteinsäureanhydriden mit Ammoniak, Monoaminen, Polyaminen, Monoalkoholen und Polyolen. Zur Derivatisierung bevorzugte Polyamine umfassen Ethylendiamin, Diethylentriamin, Triethylentetramin, Tetraethylenpentamin, Propylendiamin, etc. Geeignete Alkohole umfassen einwertige Alkohole, wie Ethanol, Allylalkohol, Dodecanol und Benzylalkohol, mehrwertige Alkohole, wie Ethylenglykol, Diethylenglykol, Propylenglykol, 1,2-Butandiol, Neopentylglykol, Glycerin, Trimethylolpropan, Erythrit, Pentaerythrit, Mannitol und Sorbitol.

Als Zusatzstoffe geeignete Bernsteinsäureanhydrid-Derivate (A) sind beispielsweise in der US 3 522 179, US 4 234 435, US 4 849 572, US 4 904 401, US 5 569 644 und US 6 165 235 beschrieben.

Bevorzugte Zusatzstoffe (B) sind Polyalkenylthiophosphonatester. Der Polyalkenylrest diese Ester weist vorzugsweise ein zahlenmittleres Molekulargewicht im Bereich von etwa 300 bis 5000, besonders bevorzugt 400 bis 2000 und insbesondere 500 bis 1500 auf. Der Polyalkenylrest leitet sich vorzugsweise von Polyolefinen ab, wie sie zuvor bei der Komponente (A) als langkettiger Kohlenwasserstoffrest beschrieben wurden. Dabei handelt es sich speziell um Polyalkenylreste, die sich von konventionellen oder reaktiven Polyisobutenen ableiten. Geeignete Verfahren zur Herstellung geeigneter Polyalkenylthiophosphonatester durch Umsetzung eines Polyolefins mit einem Thiophosphorylierungsmittel sind z. B. in der US 5 725 611 beschrieben.

Bevorzugte Zusatzstoffe (C) sind Mannich-Addukte. Derartige Addukte werden prinzipiell durch Mannich-Umsetzung von aromatischen Hydroxylverbindungen, insbesondere Phenol und Phenolderivaten, mit Aldehyden und Mono- oder Polyaminen erhalten. Vorzugsweise handelt es sich um die Umsetzungsprodukte von Polyisobuten-substituierten Phenolen mit Formaldehyd und Mono- oder Polyaminen wie Ethylendiamin, Diethylentriamin, Triethylentetramin, Tetraethylenpentamin, Dimethylaminopropylamin, etc. Geeignete Mannich-Addukte und Verfahren zu ihrer Herstellung sind z. B. in der US 5 876 468, EP-A 831 141, EP-A 1 233 990 und EP-A 1 226 188 beschrieben.

Die erfindungsgemäße Turbinenkraftstoffzusammensetzung enthält die beschriebenen mehrkernigen phenolischen Verbindungen in einer Menge von üblicherweise 0,0001 bis 1 Gew.-%, vorzugsweise 0,001 bis 0,5 Gew.-%, insbesondere 0,01 bis 0,2 Gew.-% und vor allem 0,01 bis 0,1 Gew.-%, bezogen auf die Gesamtmenge der Turbinenkraftstoffzusammensetzung.

Die Zusatzstoffe (A) bis (C) sowie gegebenenfalls weitere der zuvor genannten Zusatzstoffe können üblicherweise jeweils in Mengen von jeweils 0,0001 bis 1 Gew.-%, vorzugsweise 0,001 bis 0,6 Gew.-% und insbesondere 0,0015 bis 0,4 Gew.-%, bezogen auf die Gesamtmenge der Turbinenkraftstoffzusammensetzung, eingesetzt werden.

Weiterhin offenbart ist ein Additivkonzentrat für Turbinenkraftstoffe (jet fuels), welches wenigstens eine der beschriebenen mehrkernigen phenolischen Verbindungen sowie gegebenenfalls wenigstens ein Verdünnungsmittel sowie gegebenenfalls mindestens einen weiteren Zusatzstoff, der vorzugsweise unter den zuvor beschriebenen ausgewählt ist, enthält. In einer bevorzugten Ausführungsform enthält das erfindungsgemäße Additivkonzentrat, wie dann auch die erfindungsgemäße Turbinenkraftstoffzusammensetzung, einen oder mehrere Zusatzstoffe aus der Gruppe (A), (B) und (C), insbesondere auch Mischungen hieraus wie (A) + (B), (A) + (C), (B) + (C) und (A) + (B) + (C).

Geeignete Verdünnungsmittel sind beispielsweise bei der Erdölverarbeitung anfallende Fraktionen wie Kerosin, Naphtha oder mineralische Grundöle. Geeignet sind darüber hinaus aromatische und aliphatische Kohlenwasserstoffe wie Solvent Naphtha schwer, Solvesso® oder Shellsol® sowie Gemische dieser Lösungs- und Verdünnungsmittel.

Die beschriebenen mehrkernigen phenolischen Verbindungen liegen im Additivkonzentrat vorzugsweise in einer Menge von 0,1 bis 100 Gew.-%, besonders bevorzugt von 1 bis 80 Gew.-% und insbesondere von 10 bis 70 Gew.-%, bezogen auf das Gesamtgewicht des Konzentrats, vor.

Die beschriebenen mehrkernigen phenolischen Verbindungen eignen sich weiterhin in vorteilhafter Weise als Stabilisatoren in Ottokraftstoffen und in Mitteldestillat-Kraftstoffen, hier insbesondere in Dieselkraftstoff und Heizöl. Hierunter ist auch ihre Wirkungsweise als Antioxidantien im herkömmlichen Sinne zu verstehen. Insbesondere dienen sie über ihre Wirkungsweise als Stabilisatoren zur Verbesserung der thermischen Stabilität von Ottokraftstoffen und Mitteldestillat-Kraftstoffen. Weiterhin verhindern sie insbesondere auch über ihre Wirkungsweise als Stabilisatoren, d.h. in ihrer Eigenschaft als Dispergatoren, Ablagerungen im Kraftstoffsystem und/oder Verbrennungssystem von Otto- bzw. Dieselmotoren.

Als Ottokraftstoffe kommen alle handelsüblichen Ottokraftstoffzusammensetzungen in Betracht. Als typischer Vertreter soll hier der marktübliche Eurosuper Grundkraftstoff gemäß EN 228 genannt werden. Weiterhin sind auch Ottokraftstoffzusammensetzungen der Spezifikation gemäß WO 00/47698 mögliche Einsatzgebiete für die vorliegende Erfindung.

Als Mitteldestillat-Kraftstoffe kommen alle handelsüblichen Dieselkraftstoff- und Heizölzusammensetzungen in Betracht. Bei Dieselkraftstoffen handelt es sich üblicherweise um Erdölraffinate, die in der Regel einen Siedebereich von 100 bis 400°C haben. Dies sind meist Destillate mit einem 95%-Punkt bis zu 360°C oder auch darüber hinaus. Dies können aber auch sogenannte "Ultra low sulfur diesel" oder "City diesel" sein, gekennzeichnet durch einen 95%-Punkt von beispielsweise maximal 345°C und einem Schwefelgehalt von maximal 0,005 Gew.-% oder durch einen 95%-Punkt von beispielsweise 285°C und einem Schwefelgehalt von maximal 0,001 Gew.-%. Neben den durch Raffination erhältlichen Dieselkraftstoffen, deren Hauptbestandteile längerkettige Paraffine darstellen, sind solche, die durch Kohlevergasung oder Gasverflüssigung ["gas to liquid" (GTL) Kraftstoffe] erhältlich sind, geeignet. Geeignet sind auch Mischungen der vorstehend genannten Dieselkraftstoffe mit regenerativen Kraftstoffen wie Biodiesel oder Bioethanol. Von besonderem Interesse sind gegenwärtig Dieselkraftstoffe mit niedrigem Schwefelgehalt, das heißt mit einem Schwefelgehalt von weniger als 0,05 Gew.-%, vorzugsweise von weniger als 0,02 Gew.-%, insbesondere von weniger als 0,005 Gew.-% und speziell von weniger als 0,001 Gew.-% Schwefel. Dieselkraftstoffe können auch Wasser, z.B. in einer Menge bis zu 20 Gew.-%, enthalten, beispielsweise in Form von Diesel-Wasser-Mikroemulsionen oder als sogenannter "White Diesel".

Bei Heizölen handelt es sich beispielsweise um schwefelarme oder schwefelreiche Erdölraffinate oder um Stein- oder Braunkohledestillate, die üblicherweise einen Siedebereich von 150 bis 400°C aufweisen. Bei Heizölen kann es sich um Standard-Heizöl gemäß DIN 51603-1 handeln, das einen Schwefelgehalt von 0,005 bis 0,2 Gew.-% besitzt, oder es handelt sich um schwefelarme Heizöle mit einem Schwefelgehalt von 0 bis 0,005 Gew.-%. Als Beispiele für Heizöl sei insbesondere Heizöl für häusliche Ölfeuerungsanlagen oder Heizöl EL genannt.

Die beschriebenen mehrkernigen phenolischen Verbindungen können entweder dem jeweiligen Grundkraftstoff, insbesondere dem Otto- oder dem Dieselkraftstoff, allein oder in Form von Kraftstoffadditiv-Paketen, z.B. den sogenannten Diesel-Performance-Paketen, zugesetzt werden. Derartige Pakete stellen Kraftstoffadditiv-Konzentrate dar und enthalten in der Regel neben Lösungsmitteln noch eine Reihe weiterer Komponenten als Coadditive, beispielsweise Trägeröle, Kaltfließverbesserer, Korrosionsinhibitoren, Demulgatoren, Dehazer, Antischaummittel, weitere Cetanzahlverbesserer, weitere Verbrennungsverbesserer, Antioxidantien oder Stabilisatoren, Antistatika, Metallocene, Metalldeaktivatoren, Lösungsvermittler, Marker und/oder Farbstoffe.

Erfindungsgemäß umfasst der additivierte Otto- oder Dieselkraftstoff neben den beschriebenen mehrkernigen phenolischen Verbindungen als weitere Kraftstoffadditive inbesondere mindestens ein Detergens, nachfolgend als Komponente (D) bezeichnet.

Als Detergentien oder Detergens-Additive (D) werden üblicherweise Ablagerungsinhibitoren für Kraftstoffe bezeichnet. Vorzugsweise handelt es sich bei den Detergentien um amphiphile Substanzen, die mindestens einen hydrophoben Kohlenwasserstoffrest mit einem zahlengemittelten Molekulargewicht (Mₙ) von 85 bis 20.000, insbesondere von 300 bis 5000, vor allem von 500 bis 2500, und mindestens eine polare Gruppierung besitzen, die ausgewählt ist unter
(Da) Mono- oder Polyaminogruppen mit bis zu 6 Stickstoffatomen, wobei mindestens ein Stickstoffatom basische Eigenschaften hat;
(Db) Nitrogruppen, gegebenenfalls in Kombination mit Hydroxylgruppen;
(Dc) Hydroxylgruppen in Kombination mit Mono- oder Polyaminogruppen, wobei min destens ein Stickstoffatom basische Eigenschaften hat;
(Dd) Carboxylgruppen oder deren Alkalimetall- oder Erdalkalimetallsalzen;
(De) Sulfonsäuregruppen oder deren Alkalimetall- oder Erdalkalimetallsalzen;
(Df) Polyoxy-C₂-C₄-alkylengruppierungen, die durch Hydroxylgruppen, Mono- oder Polyaminogruppen, wobei mindestens ein Stickstoffatom basische Eigenschaften hat, oder durch Carbamatgruppen terminiert sind;
(Dg) Carbonsäureestergruppen;
(Dh) aus Bernsteinsäureanhydrid abgeleiteten Gruppierungen mit Hydroxy- und/oder Amino- und/oder Amido- und/oder Imidogruppen; und/oder
(Di) durch Mannich-Umsetzung von substituierten Phenolen mit Aldehyden und Mo no- oder Polyaminen erzeugten Gruppierungen.

Der hydrophobe Kohlenwasserstoffrest in den obigen Detergens-Additiven, welcher für die ausreichende Löslichkeit in der Brennstoffölzusammensetzung sorgt, hat ein zahlengemitteltes Molekulargewicht (Mₙ) von 85 bis 20.000, insbesondere von 300 bis 5000, vor allem von 500 bis 2500. Als typischer hydrophober Kohlenwasserstoffrest, insbesondere in Verbindung mit den polaren Gruppierungen (Da), (Dc), (Dh) und (Di), kommen längerkettige Alkyl- oder Alkenylgruppen, insbesondere der Polypropenyl-, Polybutenyl- und Polyisobutenylrest mit jeweils Mₙ = 300 bis 5000, insbesondere 500 bis 2500, vor allem 700 bis 2300, in Betracht.

Als Beispiele für obige Gruppen von Detergens-Additiven seien die folgenden genannt:
Mono- oder Polyaminogruppen (Da) enthaltende Additive sind vorzugsweise Polyalkenmono- oder Polyalkenpolyamine auf Basis von Polypropen oder konventionellem (d.h. mit überwiegend mittenständigen Doppelbindungen) Polybuten oder Polyisobuten mit Mₙ = 300 bis 5000. Geht man bei der Herstellung der Additive von Polybuten oder Polyisobuten mit überwiegend mittenständigen Doppelbindungen (meist in der β- und γ -Position) aus, bietet sich der Herstellweg durch Chlorierung und anschließende Aminierung oder durch Oxidation der Doppelbindung mit Luft oder Ozon zur Carbonyl- oder Carboxylverbindung und anschließende Aminierung unter reduktiven (hydrierenden) Bedingungen an. Zur Aminierung können hier Amine, wie z. B. Ammoniak, Monoamine oder Polyamine, wie Dimethylaminopropylamin, Ethylendiamin, Diethylentriamin, Triethylentetramin oder Tetraethylenpentamin, eingesetzt werden. Entsprechende Additive auf Basis von Polypropen sind insbesondere in der WO-A-94/24231 beschrieben.

Weitere bevorzugte Monoaminogruppen (Da) enthaltende Additive sind die Hydrierungsprodukte der Umsetzungsprodukte aus Polyisobutenen mit einem mittleren Polymerisationsgrad P = 5 bis 100 mit Stickoxiden oder Gemischen aus Stickoxiden und Sauerstoff, wie sie insbesondere in WO-A-97/03946 beschrieben sind.

Weitere bevorzugte Monoaminogruppen (Da) enthaltende Additive sind die aus Polyisobutenepoxiden durch Umsetzung mit Aminen und nachfolgender Dehydratisierung und Reduktion der Aminoalkohole erhältlichen Verbindungen, wie sie insbesondere in DE-A-196 20 262 beschrieben sind.

Nitrogruppen (Db), gegebenenfalls in Kombination mit Hydroxylgruppen, enthaltende Additive sind vorzugsweise Umsetzungsprodukte aus Polyisobutenen des mittleren Polymerisationsgrades P = 5 bis 100 oder 10 bis 100 mit Stickoxiden oder Gemischen aus Stickoxiden und Sauerstoff, wie sie insbesondere in WO-A-96/03367 und WO-A-96/03479 beschrieben sind. Diese Umsetzungsprodukte stellen in der Regel Mischungen aus reinen Nitropolyisobutenen (z. B. α,β-Dinitropolyisobuten) und gemischten Hydroxynitropolyisobutenen (z. B. α-Nitro-β-hydroxypolyisobuten) dar.

Hydroxylgruppen in Kombination mit Mono- oder Polyaminogruppen (Dc) enthaltende Additive sind insbesondere Umsetzungsprodukte von Polyisobutenepoxiden, erhältlich aus vorzugsweise überwiegend endständige Doppelbindungen aufweisendem Polyisobuten mit Mₙ = 300 bis 5000, mit Ammoniak, Mono- oder Polyaminen, wie sie insbesondere in EP-A-476 485 beschrieben sind.

Carboxylgruppen oder deren Alkalimetall- oder Erdalkalimetallsalze (Dd) enthaltende Additive sind vorzugsweise Copolymere von C₂-C₄₀-Olefinen mit Maleinsäureanhydrid mit einer Gesamt-Molmasse von 500 bis 20000, deren Carboxylgruppen ganz oder teilweise zu den Alkalimetall- oder Erdalkalimetallsalzen und ein verbleibender Rest der Carboxylgruppen mit Alkoholen oder Aminen umgesetzt sind. Solche Additive sind insbesondere aus der EP-A-307 815 bekannt. Derartige Additive dienen hauptsächlich zur Verhinderung von Ventilsitzverschleiß und können, wie in der WO-A-87/01126 beschrieben, mit Vorteil in Kombination mit üblichen Kraftstoffdetergenzien wie Poly(iso)-butenaminen oder Polyetheraminen eingesetzt werden.

Sulfonsäuregruppen oder deren Alkalimetall- oder Erdalkalimetallsalze (De) enthaltende Additive sind vorzugsweise Alkalimetall- oder Erdalkalimetallsalze eines Sulfobernsteinsäurealkylesters, wie er insbesondere in der EP-A-639 632 beschrieben ist. Derartige Additive dienen hauptsächlich zur Verhinderung von Ventilsitzverschleiß und können mit Vorteil in Kombination mit üblichen Kraftstoffdetergenzien wie Poly(iso)butenaminen oder Polyetheraminen eingesetzt werden.

Polyoxy-C₂-C₄-alkylengruppierungen (Df) enthaltende Additive sind vorzugsweise Polyether oder Polyetheramine, welche durch Umsetzung von C₂-C₆₀-Alkanolen, C₆-C₃₀-Alkandiolen, Mono- oder Di-C₂-C₃₀-alkylaminen, C₁-C₃₀-Alkylcyclohexanolen oder C₁-C₃₀-Alkylphenolen mit 1 bis 30 mol Ethylenoxid und/oder Propylenoxid und/oder Butylenoxid pro Hydroxylgruppe oder Aminogruppe und, im Falle der Polyetheramine, durch anschließende reduktive Aminierung mit Ammoniak, Monoaminen oder Polyaminen erhältlich sind. Derartige Produkte werden insbesondere in EP-A-310 875, EP-A-356 725, EP-A-700 985 und US-A-4 877 416 beschrieben. Im Falle von Polyethern erfüllen solche Produkte auch Trägeröleigenschaften. Typische Beispiele hierfür sind Tridecanol- oder Isotridecanolbutoxylate, Isononylphenolbutoxylate sowie Polyisobutenolbutoxylate und -propoxylate sowie die entsprechenden Umsetzungsprodukte mit Ammoniak.

Carbonsäureestergruppen (Dg) enthaltende Additive sind vorzugsweise Ester aus Mono-, Di- oder Tricarbonsäuren mit langkettigen Alkanolen oder Polyolen, insbesondere solche mit einer Mindestviskosität von 2 mm²/s bei 100°C, wie sie insbesondere in DE-A-38 38 918 beschrieben sind. Als Mono-, Di- oder Tricarbonsäuren können aliphatische oder aromatische Säuren eingesetzt werden, als Esteralkohole bzw. -polyole eignen sich vor allem langkettige Vertreter mit beispielsweise 6 bis 24 C-Atomen. Typische Vertreter der Ester sind Adipate, Phthalate, iso-Phthalate, Terephthalate und Trimellitate des iso-Octanols, iso-Nonanols, iso-Decanols und des iso-Tridecanols. Derartige Produkte erfüllen auch Trägeröleigenschaften.

Aus Bernsteinsäureanhydrid abgeleitete Gruppierungen mit Hydroxy- und/oder Amino- und/oder Amido- und/oder Imidogruppen (Dh) enthaltende Additive sind vorzugsweise entsprechende Derivate von Alkyl- oder Alkenyl-substituiertem Bernsteinsäureanhydrid und insbesondere die entsprechenden Derivate von Polyisobutenylbernsteinsäureanhydrid, welche durch Umsetzung von konventionellem oder hochreaktivem Polyisobuten mit Mₙ = 300 bis 5000 mit Maleinsäureanhydrid auf thermischem Weg oder über das chlorierte Polyisobuten erhältlich sind. Von besonderem Interesse sind hierbei Derivate mit aliphatischen Polyaminen wie Ethylendiamin, Diethylentriamin, Triethylentetramin oder Tetraethylenpentamin. Bei den Gruppierungen mit Hydroxy- und/oder Amino- und/oder Amido- und/oder Imidogruppen handelt es sich beispielsweise um Carbonsäuregruppen, Säureamide von Monoaminen, Säureamide von Di- oder Polyaminen, die neben der Amidfunktion noch freie Amingruppen aufweisen, Bernsteinsäurederivate mit einer Säure- und einer Amidfunktion, Carbonsäureimide mit Monoaminen, Carbonsäureimide mit Di- oder Polyaminen, die neben der Imidfunktion noch freie Amingruppen aufweisen, oder Diimide, die durch die Umsetzung von Di- oder Polyaminen mit zwei Bernsteinsäurederivaten gebildet werden. Derartige Kraftstoffadditive sind insbesondere in US-A-4 849 572 beschrieben.

Bei den Detergens-Additiven aus der Gruppe (Dh) handelt es sich vorzugsweise um die Umsetzungsprodukte von Alkyl- oder Alkenyl-substituierten Bernsteinsäureanhydriden, insbesondere von Polyisobutenylbernsteinsäureanhydriden, mit Aminen und/oder Alkoholen. Es handelt sich hierbei somit um von Alkyl-, Alkenyl- oder Polyisobutenyl-Bernsteinsäureanhydrid abgeleitete Derivate mit Amino- und/oder Amido- und/oder Imido- und/oder Hydroxylgruppen. Es versteht sich von selbst, dass diese Umsetzungsprodukte nicht nur bei Einsatz von substituiertem Bernsteinsäureanhydrid, sondern auch bei Verwendung von substituierter Bernsteinsäure oder geeigneten Säurederivaten, wie Bernsteinsäurehalogenide oder -ester, erhältlich sind.

In einer bevorzugten Ausführungsform umfasst der additivierte Kraftstoff mindestens ein Detergens auf Basis eines Polyisobutenyl-substituierten Bernsteinsäureimids. Speziell von Interesse sind die Imide mit aliphatischen Polyaminen. Besonders bevorzugte Polyamine sind dabei Ethylendiamin, Diethylentriamin, Triethylentetramin, Pentaethylenhexamin und vor allem Tetraethylenpentamin. Der Polyisobutenylrest besitzt ein zahlenmittleres Molekulargewicht Mₙ von vorzugsweise 500 bis 5000, besonders bevorzugt von 500 bis 2000 und insbesondere von etwa 1000.

Durch Mannich-Umsetzung von substituierten Phenolen mit Aldehyden und Mono- oder Polyaminen erzeugte Gruppierungen (Di) enthaltende Additive sind vorzugsweise Umsetzungsprodukte von Polyisobuten-substituierten Phenolen mit Formaldehyd und Mono- oder Polyaminen wie Ethylendiamin, Diethylentriamin, Triethylentetramin, Tetraethylenpentamin oder Dimethylaminopropylamin. Die Polyisobutenyl-substituierten Phenole können aus konventionellem oder hochreaktivem Polyisobuten mit Mₙ = 300 bis 5000 stammen. Derartige "Polyisobuten-Mannichbasen" sind insbesondere in der EP-A-831 141 beschrieben.

Vorzugsweise werden die genannten Detergens-Additive (D) zusammen mit den beschriebenen mehrkernigen phenolischen Verbindungen in Kombination mit wenigstens einem Trägeröl verwendet.

Geeignete mineralische Trägeröle sind bei der Erdölverarbeitung anfallende Fraktionen, wie Brightstock oder Grundöle mit Viskositäten wie beispielsweise aus der Klasse SN 500 - 2000; aber auch aromatische Kohlenwasserstoffe, paraffinische Kohlenwasserstoffe und Alkoxyalkanole. Brauchbar ist ebenfalls eine als "hydrocrack oil" bekannte und bei der Raffination von Mineralöl anfallende Fraktion (Vakuumdestillatschnitt mit einem Siedebereich von etwa 360 bis 500°C, erhältlich aus unter Hochdruck katalytisch hydriertem und isomerisiertem sowie entparaffiniertem natürlichen Mineralöl). Ebenfalls geeignet sind Mischungen oben genannter mineralischer Trägeröle.

Beispiele für geeignete synthetische Trägeröle sind ausgewählt unter: Polyolefinen (Polyalphaolefinen oder Polyinternalolefinen), (Poly)estern, (Poly)alkoxylaten, Polyethern, aliphatischen Polyetheraminen, alkylphenolgestarteten Polyethern, alkylphenolgestarteten Polyetheraminen und Carbonsäureestern langkettiger Alkanole.

Beispiele für geeignete Polyolefine sind Olefinpolymerisate mit Mₙ = 400 bis 1800, vor allem auf Polybuten- oder Polyisobuten-Basis (hydriert oder nicht hydriert).

Beispiele für geeignete Polyether oder Polyetheramine sind vorzugsweise Polyoxy-C₂-C₄-alkylengruppierungen enthaltende Verbindungen, welche durch Umsetzung von C₂-C₆₀-Alkanolen, C₆-C₃₀-Alkandiolen, Mono- oder Di-C₂-C₃₀-alkylaminen, C₁-C₃₀-Alkylcyclohexanolen oder C₁-C₃₀-Alkylphenolen mit 1 bis 30 mol Ethylenoxid und/ oder Propylenoxid und/oder Butylenoxid pro Hydroxylgruppe oder Aminogruppe und, im Falle der Polyetheramine, durch anschließende reduktive Aminierung mit Ammoniak, Monoaminen oder Polyaminen erhältlich sind. Derartige Produkte werden insbesondere in EP-A 310 875, EP-A 356 725, EP-A 700 985 und US-A 4,877,416 beschrieben. Beispielsweise können als Polyetheramine Poly-C₂-C₆-Alkylenoxidamine oder funktionelle Derivate davon verwendet werden. Typische Beispiele hierfür sind Tridecanol- oder Isotridecanolbutoxylate, Isononylphenolbutoxylate sowie Polyisobutenolbutoxylate und -propoxylate sowie die entsprechenden Umsetzungsprodukte mit Ammoniak.

Beispiele für Carbonsäureester langkettiger Alkanole sind insbesondere Ester aus Mono-, Di- oder Tricarbonsäuren mit langkettigen Alkanolen oder Polyolen , wie sie insbesondere in der DE-A 38 38 918 beschrieben sind. Als Mono-, Di- oder Tricarbonsäuren können aliphatische oder aromatische Säuren eingesetzt werden, als Esteralkohole bzw. -polyole eignen sich vor allem langkettige Vertreter mit beispielsweise 6 bis 24 C-Atomen. Typische Vertreter der Ester sind Adipate, Phthalate, iso-Phthalate, Terephthalate und Trimellitate des Isooctanols, Isononanols, Isodecanols und des Isotridecanols, wie z. B. Di-(n- oder iso-tridecyl)phthalat.

Weitere geeignete Trägerölsysteme sind beispielsweise in DE-A 38 26 608, DE-A 41 42 241, DE-A 43 09 074, EP-A 0 452 328 und EP-A 0 548 617 beschrieben.

Beispiele für besonders geeignete synthetische Trägeröle sind alkoholgestartete Polyether mit etwa 5 bis 35, wie z. B. etwa 5 bis 30, C₃-C₆-Alkylenoxideinheiten, wie z. B. ausgewählt unter Propylenoxid-, n-Butylenoxid- und i-Butylenoxid-Einheiten, oder Gemischen davon. Nichtlimitierende Beispiele für geeignete Starteralkohole sind langkettige Alkanole oder mit langkettigem Alkyl-substituierte Phenole, wobei der langkettige Alkylrest insbesondere für einen geradkettigen oder verzweigten C₆-C₁₈-Alkylrest steht. Als bevorzugte Beispiele sind zu nennen Tridecanol und Nonylphenol.

Weitere geeignete synthetische Trägeröle sind alkoxylierte Alkylphenole, wie sie in der DE-A 101 02 913 beschrieben sind.

Bevorzugte Trägeröle sind synthetische Trägeröle, wobei Polyether besonders bevorzugt sind.

Dem additivierten Kraftstoff wird das Detergens-Additiv (D) oder ein Gemisch verschiedener solcher Detergens-Additive in einer Gesamtmenge von vorzugsweise 10 bis 2000 Gew.-ppm, besonders bevorzugt von 20 bis 1000 Gew.-ppm, stärker bevorzugt von 50 bis 500 Gew.-ppm und insbesondere von 50 bis 200 Gew.-ppm, z.B. von 70 bis 150 Gew.-ppm, zugesetzt.

Wenn ein Trägeröl mitverwendet wird, so wird dieses dem erfindungsgemäßen additivierten Kraftstoff in einer Menge von vorzugsweise 1 bis 1000 Gew.-ppm, besonders bevorzugt von 10 bis 500 Gew.-ppm und insbesondere von 20 bis 100 Gew.-ppm, zugesetzt.

Als weitere Coadditive geeignete Kaltfließverbesserer sind beispielsweise Copolymere von Ethylen mit wenigstens einem weiteren ungesättigten Monomer, z.B. Ethylen-Vinylacetat-Copolymere.

Als weitere Coadditive geeignete Korrosionsinhibitoren sind beispielsweise Bernsteinsäureester, vor allem mit Polyolen, Fettsäurederivate, z.B. Ölsäureester, oligomerisierte Fettsäuren und substituierte Ethanolamine.

Als weitere Coadditive geeignete Demulgatoren sind beispielsweise die Alkali- und Erdalkalimetallsalze von alkylsubstituierten Phenol- und Naphthalinsulfonaten und die Alkali- und Erdalkalimetallsalze von Fettsäure, weiterhin Alkoholalkoxylate, z.B. Alkoholethoxylate, Phenolalkoxylate, z.B. tert.-Butylphenolethoxylate oder tert.-Pentyl-phenolethoxylate, Fettsäure, Alkylphenole, Kondensationsprodukte von Ethylenoxid und Propylenoxid, z.B. Ethylenoxid-Propylenoxid-Blockcopolymere, Polyethylenimine und Polysiloxane.

Als weitere Coadditive geeignete Dehazer sind beispielsweise alkoxylierte Phenol-Formaldehyd-Kondensate.

Als weitere Coadditive geeignete Antischaummittel sind beispielsweise Polyethermodifizierte Polysiloxane.

Als weitere Coadditive geeignete Cetanzahl- und Verbrennungsverbesserer sind beispielsweise Alkylnitrate, z.B. Cyclohexylnitrat und insbesondere 2-Ethylhexylnitrat, und Peroxide, z.B. Di-tert.-butylperoxid.

Als weitere Coadditive geeignete Antioxidantien sind beispielsweise substituierte Phenole, z.B. 2,6-Di-tert.-butylphenol und 2,6-Di-tert.-butyl-3-methylphenol, sowie Phenylendiamine, z.B. N,N'-Di-sec.-butyl-p-phenylendiamin.

Als weitere Coadditive geeignete Metalldeakivatoren sind beispielsweise Salicylsäure-Derivate, z.B. N,N'-Disalicyliden-1,2-propandiamin.

Als Lösungsmittel eignen sich, insbesondere für Kraftstoffadditiv-Pakete, beispielsweise unpolare organische Lösungsmittel, insbesondere aromatische und aliphatische Kohlenwasserstoffe, z.B. Toluol, Xylole, "white spirit" sowie die technischen Lösungsmittelgemische der Bezeichnungen Shellsol® (Hersteller: Royal Dutch / Shell Group), Exxol® (Hersteller: ExxonMobil) und Solvent Naphtha. Weiterhin kommen hier, insbesondere in Abmischung mit den genannten unpolaren organischen Lösungsmitteln, polare organische Lösungsmittel, vor allem Alkohole wie 2-Ethylhexanol, Decanol und Isotridecanol, in Betracht.

Wenn die genannten Coadditive und/oder Lösungsmittel in Otto- oder Dieselkraftstoff mitverwendet werden, werden sie in den hierfür übliche Mengen eingesetzt.

Die beschriebenen mehrkernigen phenolischen Verbindungen eignen sich weiterhin in besonders vorteilhafter Weise als Stabilisatoren in Schmierstoffen. Als Schmierstoffe oder Schmierstoffzusammensetzungen sollen hier Motorenöle, Schmieröle, Getriebe-, Schalt- und Automatiköle und verwandte flüssige Zusammensetzungen, die der Schmierung von mechanisch bewegten Teilen - zumeist als Metall - dienen, bezeichnet werden. Unter Stabilisierung ist hier vor allem die Verbesserung der Oxidations- und Alterungsstabilität von Schmierstoffzusammensetzungen, also deren Wirkungsweise insbesondere als Antioxidantien, zu verstehen. Zusätzlich oder alternativ wird durch die beschriebenen mehrkernigen phenolischen Verbindungen die Scherstabiliät von Schmierstoffzusammensetzungen verbessert, d.h. die mehrkernigen phenolischen Verbindungen verdicken die Schmierstoffzusammensetzungen effektiver. In einigen Fällen wirken die beschriebenen mehrkernigen phenolischen Verbindungen auch als Dispergatoren in Schmierstoffzusammensetzungen.

Weiterhin offenbart ist eine Schmierstoffstoffzusammensetzung, die hierfür übliche Komponenten und wenigstens eine der beschriebenen mehrkernigen phenolischen Verbindungen enthält. Die erfindungsgemäße Schmierstoffzusammensetzung enthält die beschriebenen mehrkernigen phenolischen Verbindungen in einer Menge von üblicherweise 0,001 bis 20 Gew.-%, vorzugsweise 0,01 bis 10 Gew.-%, insbesondere 0,05 bis 8 Gew.-% und vor allem 0,1 bis 5 Gew.-%, bezogen auf die Gesamtmenge der Schmierstoffzusammensetzung.

Die wirtschaftlich bedeutsamsten Schmierstoffzusammensetzungen sind Motorenöle sowie Getriebe-, Schalt- und Automatiköle. Motorenöle bestehen üblicherweise aus mineralischen Grundölen, welche überwiegend paraffinische Bestandteile enthalten und durch aufwendige Aufarbeitungs- und Reinigungsprozesse in der Raffinerie hergestellt werden, mit einem Anteil an ca. 2 bis 10 Gew.-% an Additiven (bezogen auf die Wirksubstanz-Gehalte). Für spezielle Anwendungen, beispielsweise Hochtemperatur-Einsätze, können die mineralischen Grundöle teilweise oder vollständig durch synthetische Komponenten wie organische Ester, synthetische Kohlenwasserstoffe wie Olefinoligomere, Poly-α-Olefine oder Polyolefine oder Hydrocrack-Öle ersetzt sein. Motorenöle müssen auch bei hohen Temperaturen ausreichend hohe Viskositäten aufweisen, um einen einwandfreien Schmiereffekt und eine gute Abdichtung zwischen Zylinder und Kolben zu gewährleisten. Weiterhin müssen Motorenöle von ihren Fließeigenschaften auch so beschaffen sein, dass bei niedrigen Temperaturen der Motor problemlos gestartet werden kann. Motorenöle müssen oxidationsstabil sein und dürfen auch unter schweren Arbeitsbedingungen nur wenig Zersetzungsprodukte in flüssiger oder fester Form sowie Ablagerungen erzeugen. Motorenöle dispergieren Feststoffe (Dispersant-Verhalten), verhindern Ablagerungen (Detergent-Verhalten), neutralisieren saure Reaktionsprodukte und bilden einen Verschleißschutzfilm auf den Metalloberflächen im Motor aus. Motorenöle werden üblicherweise nach Viskositätsklassen-Klassen (SAE-Klassen) charakterisiert.

Getriebe-, Schalt- und Automatiköle sind bezügliche ihrer Grundkomponenten und Additive ähnlich wie Motorenöle zusammengesetzt. Die Kraftübertragung im Zahnradsystem von Getrieben erfolgt zu einem hohen Anteil durch den Flüssigkeitsdruck im Getriebeöl zwischen den Zähnen. Das Getriebeöl muß demzufolge so beschaffen sein, dass es auf Dauer hohe Drücke aushält, ohne sich zu zersetzen. Neben den Viskositätseigenschaften sind hier Verschleiß, Druckfestigkeit, Reibung, Scherstabilität, Traktion und Einlaufverhalten die entscheidenden Größen.

Motorenöle und Getriebe-, Schalt- und Automatiköle enthalten neben den im Sinne der vorliegenden Erfindung einzusetzenden mehrkernigen phenolischen Verbindungen in der Regel noch mindestens eines, meist jedoch einige oder alle der nachfolgend aufgeführten Additive in den hierfür in der Regel üblichen Mengen (welche in Gew.-%, bezogen auf die Gesamt-Schmierstoffzusammensetzung, in Klammern angegeben sind):
(a) Antioxidantien (0,1 bis 5 %):
   Schwefelverbindungen, z.B. Reaktionsprodukte von Terpenen (α-Pinen), Harzölen oder niedermolekularen Polybutenen mit Schwefel, Dialkylsulfide, Dialkyltrisulfide, Polysulfide, Diarylsulfide, modifizierte Thiole, Mercaptobenzimidazole, Mercaptotriazine, Thiophen-Derivate, Xanthate, Zink-dialkyldithiocarbamate, Thioglykole, Thioaldehyde, Dibenzyldisulfid, Alkylphenolsulfide, Dialkylphenolsulfide oder schwefelhaltige Carbonsäuren
   Phosphorverbindungen, z.B. Triaryl- und Trialkylphosphite, 3,5-di-tert.-butyl-4-hydroxybenzyl-phosphonsäure-dialkylester oder Phosphonsäure-piperazide
   Schwefel-Phosphor-Verbindungen, z.B. Zink-dialkyldithiophosphate (Metalldialkyldithiophosphate wirken in Schmierölen auch als Korrosionsinhibitoren und Hochdruckadditive) oder Reaktionsprodukte von Phosphorpentasulfid mit Terpenen (α-Pinen, Dipenten), Polybutenen, Olefinen oder ungesättigten Estern
   Phenol-Derivate, z.B. sterisch gehinderte Mono-, Bis- oder Trisphenole, sterisch gehinderte mehrkernige Phenole, Polyalkylphenole, 2,6-Di-tert.-butyl-4-methylphenol oder Methylen-4,4'-bis(2,6-di-tert.-butylphenol) (Phenol-Derivate werden oft in Kombination mit Antioxidantien auf Schwefelbasis oder Aminbasis eingesetzt) Amine, z.B. Arylamine wie Diphenylamin, Phenyl-α-naphthylamin oder 4,4'-Tetramethyldiaminodiphenylmethan
   Metalldeaktivatoren im engeren Sinne, z.B. N-Salicyliden-ethylamin, N,N'-Disalicyliden-ethylendiamin, N,N'-Disalicyliden-1,2-propandiamin, Triethylendiamin, Ethylendiamintetraessigsäure, Phosphorsäure, Zitronensäure, Glykolsäure, Lecithin, Thiadiazol, Imidazol oder Pyrazol-Derivate
(b) Viskositätsindex-Verbesserer (0,05 bis 10 %), z.B: Polyisobutene mit einem Molekulargewicht von üblicherweise 10.000 bis 45.000, Polymethacrylate mit einem Molekulargewicht von üblicherweise15.000 bis 100.000, Homo- und Copolymerisate von 1,3-Dienen wie Butadien oder Isopren mit einem Molekulargewicht von üblicherweise 80.000 bis 100.000, 1,3-Dien-Styrol-Copolymerisate mit einem Molekulargewicht von üblicherweise 80.000 bis 100.000, Maleinsäureanydrid-Styrol-Polymere in veresterter Form mit einem Molekulargewicht von üblicherweise 60.000 bis 120.000, sternförmige Polymere mit blockförmigem Aufbau durch Einheiten aus konjugierten Dienen und aromatischen Monomeren mit einem Molekulargewicht von üblicherweise 200.000 bis 500.000, Polyalkylstyrole mit einem Molekulargewicht von üblicherweise 80.000 bis 150.000, Polyolefine aus Ethylen und Propylen oder Styrol-Cyclopentadien-Norbornen-Terpolymere mit einem Molekulargewicht von üblicherweise 60.000 bis 140.000
(c) Pour Point Erniedriger (Kaltfließverbesserer) (0,03 bis 1 %), z.B. bicyclische Aromaten wie Naphthalin mit verschiedenen langkettigen Alkylresten, Polymethyacrylate mit 12 bis 18 Kohlenstoffatomen im Alkoholrest, einem Verzweigungsgrad zwischen 10 bis 30 mol-% und einem durchschnittlichen Molekulargewicht von 5.000 bis 500.000, langkettige Alkylphenole und Phthalsäure-dialkylarylester oder Copolymere verschiedener Olefine
(d) Detergentien (HD Additive) (0,2 bis 4 %), z.B. Calcium-, Blei-, Zink- und Mangan-Naphthenate, Calcium-Dichlorostearate, Calcium-Phenylstearate, Calcium-Chlorophenylstearate, Sulfonierungsprodukte von Alkylaromaten wie Dodecylbenzol, Petroleumsulfonate, Natrium-, Calcium-, Barium- oder Magnesium-Sulfonate, neutrale, basische und überbasische Sulfonate, Phenate und Carboxylate, Salicylate, Metallsalze von Alkylphenolen und Alkylphenolsulfiden, Phosphate, Thiophosphate oder Alkenylphosphonsäure-Derivate
(e) Aschefreie Dispersantien (0,5 bis 10 %), z.B. Mannich-Kondensate aus Alkylphenol, Formaldehyd und Polyalkylenpolyaminen, Umsetzungsprodukte von Polyisobutenylsuccinanhydriden mit Polyhydroxy-Verbindungen oder Polyaminen, Copolymerisate von Alkylmethacrylaten mit Diethylaminoethyl-methacrylat, N-Vinylpyrrolidon, N-Vinylpryridin oder 2-Hydroxyethyl-methacrylat oder Vinylacetat-Fumarat-Copolymerisate
(f) Hochdruckadditive (Extreme Pressure Additive) (0,2 bis 2,5 %), z.B. chlorierte Paraffine mit 40 bis 70 Gew.-% Chlorgehalt, chlorierte Fettsäure (insbesondere mit Trichlormethyl-Endgruppen), Dialkylhydrogenphosphite, Triarylphosphite, Arylphosphate wie Trikresylphosphat, Dialkylphosphate, Trialkylphosphate wie Tributylphosphat, Trialkylphosphine, Diphosphorsäureester, Nitroaromaten, Aminophenol-Derivate der Naphthensäure, Carbaminsäureester, Dithiocarbaminsäure-Derivate, substituierte 1,2,3-Triazole, Mischungen aus Benzotriazol und Alkylbernsteinsäureanhydrid oder Alkylmaleinsäureanhydrid, 1,2,4-Thiadiazol-Polymere, Morpholinobenzothiadiazoldisulfid, chlorierte Alkylsulfide, sulfurisierte Olefine, sulfurisierte Chlornaphthaline, chlorierte Alkylthiocarbonate, organische Sulfide und Polysulfide wie Bis(4-chlorbenzyl)-disulfid und Tetrachlordiphenylsulfid, Trichloracrolein-Mercaptale oder insbesondere Zink-dialkyldithiophosphate (ZDDP)
(g) Reibungsverminderer (Friction Modifier) (0,05 bis 1 %), insbesondere polare öllösliche Verbindungen, die eine dünne Schicht auf der Reibungsfläche durch Adsorption erzeugen, z.B. Fettalkohole, Fettamide, Fettsäuresalze, Fettsäurealkylester oder Fettsäureglyceride
(h) Antischaum-Additive (0,0001 bis 0,2 %), z.B. flüssige Silicone wie Polydimethylsiloxane oder Polhethylenglykolether und -sulfide
(i) Demulgatoren (0,1 bis 1 %), z.B. Dinonylnaphthalinsulfonate in Form ihrer Alkali- und Erdalkalimetallsalze
(j) Korrosionsinhibitoren (auch als Metalldeaktivatoren bezeichnet) (0,01 bis 2 %), z.B. tertiäre Amine und ihre Salze, Iminoester, Amidoxime, Diaminomethane, Derivate von gesättigten oder ungesättigten Fettsäuren mit Alkanolaminen, Alkylamine, Sarcosine, Imidazoline, Alkylbenzotriazole, Dimercaptothiadiazol-Derviate, Diarylphosphate, Thiophosphorsäureester, Neutralsalze von primären n-C₈-C₁₈-Alkylaminen oder Cycloalkylaminen mit Dialkylphosphaten mit verzweigten C₅-C₁₂-Alkylgruppen, neutrale oder basische Erdalkalimetallsulfonate, Zinknaphthenate, Mono- und Dialkylarylsulfonate, Barium-dinonylnaphthalinsulfonate, Lanolin (Wollfett), Schwermetallsalze der Naphthensäure, Dicarbonsäure, ungesättigte Fettsäuren, Hydroxyfettsäuren, Fettsäureester, Pentaerythritol- und Sorbitan-Monooleate, O-Stearoylalkanolamine, Polyisobutenylbernsteinsäure-Derivate oder Zink-dialkyldithiophosphate und Zinkdialkyldithiocarbamate
(k) Emulgatoren (0,01 bis 1 %), z.B. langkettige ungesättigte, natürlich vorkommmende Carbonsäure, Naphthensäuren, synthetische Carbonsäure, Sulfonamide, N-Oleylsarcosin, Alkansulfamidoessigsäure, Dodecylbenzolsulfonat, langkettige alkylierte Ammoniumsalze wie Dimethyldodecylbenzylammonium-chlorid, Imidazolinium-Salze, Alkyl-, Alkylaryl-, Acyl-, Alkylamino- und Acylaminopolyglykole oder langkettige acylierte Mono- und Diethanolamine
(l) Farbstoffe und Fluoreszenzadditive (0,001 bis 0,2 %)
(m) Konservierungsmittel (0,001 bis 0,5 %)
(n) Geruchsverbesserer (0,001 bis 0,2 %)

Typische gebrauchsfertige Motorenöl- und Getriebe-, Schalt- und Automatiköl-Zusammensetzungen im Rahmen der vorliegenden Erfindung sind folgendermaßen zusammengesetzt, wobei sich die Angaben für die Additive auf die Wirksubstanz-Gehalte beziehen und die Summe aller Komponenten immer 100 Gew.-% ergibt:
- 80 bis 99,3 Gew.-%, insbesondere 90 bis 98 Gew.-% Motorenöl- bzw. Getriebe-, Schalt- und Automatiköl-Grundlage (mineralische Grundöle und/oder synthetische Komponenten) einschließlich der Anteile an Lösungs- und Verdünnungsmittel für die Additive
- 0,1 bis 8 Gew.-% mehrkernige phenolische Verbindungen als Stabilisatoren
- 0,2 bis 4 Gew.-%, insbesondere 1,3 bis 2,5 Gew.-% Detergentien der Gruppe (d)
- 0,5 bis 10 Gew.-%, insbesondere 1,3 bis 6,5 Gew.-% Dispersantien der Gruppe (e)
- 0,1 bis 5 Gew.-% ,insbesondere 0,4 bis 2,0 Gew.-% Antioxidantien der Gruppe (a) und/oder Hochdruckadditive der Gruppe (f) und/oder Reibungsverminderer der Gruppe (g)
- 0,05 bis 10 Gew.-%, insbesondere 0,2 bis 1,0 Gew.-% Viskositätsindex-Verbesserer der Gruppe (b)
- 0 bis 2 Gew.-% der sonstigen Additive der Gruppen (c) und (h) bis (n)

Weiterhin offenbart ist auch ein Verfahren zur Herstellung der beschriebenen mehrkernigen phenolischen Verbindungen, welches dadurch gekennzeichnet ist, dass man das Tetrahydrobenzoxazin I mit einem oder mehreren der gleichen oder verschiedener Phenole II und/oder mit einem oder mehreren der gleichen oder verschiedener Tetrahydrobenzoxazine I bei Temperaturen von 60 bis 250°C, insbesondere 90 bis 150°C, in Abwesenheit oder in Gegenwart eines inerten Lösungsmittels, beispielsweise einem aromatischen Kohlenwasserstoff wie Toluol oder Xylol, umsetzt.

Mehrkernige phenolische Verbindungen mit mindestens einem längerkettigen Hydrocarbylrest mit mindestens 26 Kohlenstoffatomen sind offenbart. Mehrkernige phenolische Verbindungen mit bis zu 20 Benzolkernen pro Molekül, welche durch Umsetzung eines Tetrahydrobenzoxazins der allgemeinen Formel I 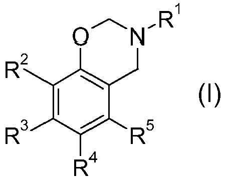 in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet,
wobei R⁶ ein Wasserstoffatom oder einen C₁- bis C₄-Alkylrest bezeichnet, und
in der die Substituenten R², R³, R⁴ und R⁵ unabhängig voneinander für Wasserstoffatome, Hydroxylgruppen oder Hydrocarbylreste mit jeweils 1 bis 3000 Kohlenstoffatomen, stehen, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
mit einem oder mehreren der gleichen oder verschiedener Phenole der allgemeinen Formel II 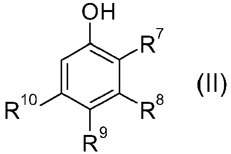 in der die Substituenten R⁷, R⁸, R⁹ und R¹⁰ unabhängig voneinander für Wasserstoffatome, Hydroxylgruppen oder Hydrocarbylreste mit jeweils 1 bis 3000 Kohlenstoffatomen, stehen, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
und/oder mit einem oder mehreren der gleichen oder verschiedener Tetrahydrobenzoxazine der allgemeinen Formel I,
wobei der Substituent R⁴ auch für einen Rest der Formel Z und der Substituent R⁹ auch für einen Rest der Formel Z' stehen kann 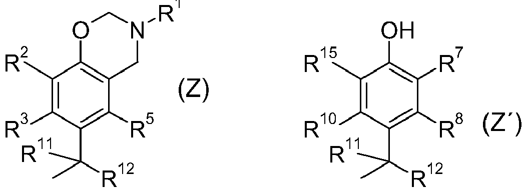 in denen die Substituenten R¹, R², R³, R⁵, R⁷, R⁸ und R¹⁰ die vorgenannten Bedeutungen haben, der Substituent R⁷ auch ein aus einem Tetrahydrobenzoxazin der allgemeinen Formel I abgeleiteter Rest sein kann, der Sustituent R¹⁵ für Wasserstoff oder einen aus einem Tetrahydrobenzoxazin der allgemeinen Formel I abgeleiteten Rest steht und die Substituenten R¹¹ und R¹² gleich oder verschieden sein können und Wasserstoff oder einen C₁- bis C₁₀-Alkylrest bezeichnen,
und in der die Substituenten R² und R³ oder R³ und R⁴ oder R⁴ und R⁵ mit der an den Benzolkern angebundenen Teilstruktur -O-CH₂-NR¹³-CH₂- auch einen zweiten Tetrahydrooxazin-Ring oder die Substituenten R² und R³ und R⁴ und R⁵ mit den an den Benzolkern angebundenen Teilstrukturen -O-CH₂-NR¹³-CH₂- und -O-CH₂-NR¹⁴-CH₂- auch einen zweiten und einen dritten Tetrahydrooxazin-Ring ausbilden können, wobei R¹³ und R¹⁴ unabhängig voneinander Hydrocarbylreste mit jeweils 1 bis 3000 Kohlenstoffatomen bedeutet, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
mit der Maßgabe, dass mindestens einer der Substituenten R¹, R², R³, R⁴, R⁵, R⁷, R⁸, R9, R¹⁰, R¹³ oder R¹⁴ 26 bis 3000 Kohlenstoffatome aufweist und die übrigen Substituenten aus der Gruppe R¹, R², R³, R⁴, R⁵, R⁷, R⁸, R⁹, R¹⁰, R¹³ oder R¹⁴, wenn sie für Hydrocarbylreste stehen, jeweils 1 bis 20 Kohlenstoffatome aufweisen, sind erhältlich.

Mehrkernige phenolische Verbindungen mit mindestens einem längerkettigen Hydrocarbylrest mit im Mittel mindestens 13 Kohlenstoffatomen, der sich von Oligomeren oder Polymeren von C₂- bis C₁₂-Olefinen ableitet, sind offenbart. Mehrkernige phenolische Verbindungen mit bis zu 20 Benzolkernen pro Molekül, welche durch Umsetzung eines Tetrahydrobenzoxazins der allgemeinen Formel I 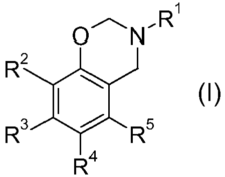 in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet, wobei R⁶ ein Wasserstoffatom oder einen C₁- bis C₄-Alkylrest bezeichnet, und
in der die Substituenten R², R³, R⁴ und R⁵ unabhängig voneinander für Wasserstoffatome, Hydroxylgruppen oder Hydrocarbylreste mit jeweils 1 bis 3000 Kohlenstoffatomen, stehen, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
mit einem oder mehreren der gleichen oder verschiedener Phenole der allgemeinen Formel II 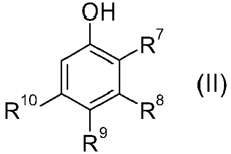 in der die Substituenten R⁷, R⁸, R⁹ und R¹⁰ unabhängig voneinander für Wasserstoffatome, Hydroxylgruppen oder Hydrocarbylreste mit jeweils 1 bis 3000 Kohlenstoffatomen, stehen, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
und/oder mit einem oder mehreren der gleichen oder verschiedener Tetrahydrobenzoxazine der allgemeinen Formel I,
wobei der Substituent R⁴ auch für einen Rest der Formel Z und der Substituent R⁹ auch für einen Rest der Formel Z' stehen kann 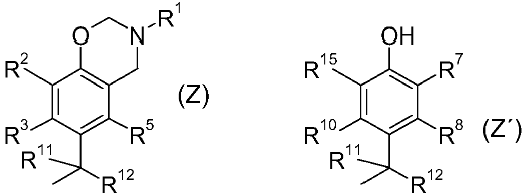 in denen die Substituenten R¹, R², R³, R⁵, R⁷, R⁸ und R¹⁰ die vorgenannten Bedeutungen haben, der Substituent R⁷ auch ein aus einem Tetrahydrobenzoxazin der allgemeinen Formel I abgeleiteter Rest sein kann, der Sustituent R¹⁵ für Wasserstoff oder einen aus einem Tetrahydrobenzoxazin der allgemeinen Formel I abgeleiteten Rest steht und die Substituenten R¹¹ und R¹² gleich oder verschieden sein können und Wasserstoff oder einen C₁- bis C₁₀-Alkylrest bezeichnen,
und in der die Substituenten R² und R³ oder R³ und R⁴ oder R⁴ und R⁵ mit der an den Benzolkern angebundenen Teilstruktur -O-CH₂-NR¹³-CH₂- auch einen zweiten Tetrahydrooxazin-Ring oder die Substituenten R² und R³ und R⁴ und R⁵ mit den an den Benzolkern angebundenen Teilstrukturen -O-CH₂-NR¹³-CH₂- und -O-CH₂-NR¹⁴-CH₂- auch einen zweiten und einen dritten Tetrahydrooxazin-Ring ausbilden können, wobei R¹³ und R¹⁴ unabhängig voneinander Hydrocarbylreste mit jeweils 1 bis 3000 Kohlenstoffatomen bedeutet, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
mit der Maßgabe, dass mindestens einer der Substituenten R¹, R², R³, R⁴, R⁵, R⁷, R⁸, R9, R¹⁰, R¹³ oder R¹⁴ im Mittel 13 bis 3000 Kohlenstoffatome aufweist und sich von Oligomeren oder Polymeren von C₂- bis C₁₂-Olefinen ableitet und die übrigen Substituenten aus der Gruppe R¹, R², R³, R⁴, R⁵, R⁷, R⁸, R⁹, R¹⁰, R¹³ oder R¹⁴, wenn sie für Hydrocarbylreste stehen, jeweils 1 bis 20 Kohlenstoffatome aufweisen, sind erhältlich. Derartige meist polydisperse Hydrocarbylreste mit polymerer Verteilung, die im Mittel 13 bis 3000 Kohlenstoffatome aufweisen und sich von Oligomeren oder Polymeren von C₂- bis C₁₂-Olefinen ableiten, sind beispielsweise solche, die sich von Ethylen, Propylen, Buten, Isobuten, Styrol, Methylstyrol, Hexen-1, Octen-1, Decen-1 oder Dodecen-1 ableiten. Sie können Homo- oder Copolymer-Reste sein. Ihr zahlenmittleres Molekulargewicht Mₙ beträgt mindestens 183, ihr Polydispersitätsindex PDI üblicherweise 1,05 bis 10. Bei niedermolekularen Resten mit Mₙ von 183 bis ca. 500 können sie auch monodispers vorliegen. Von besonderem Interesse sind polydisperse Hydrocarbylreste mit polymerer Verteilung, die sich von Isobuten ableiten.

Weiterhin werden Oligo- und Polytetrahydrobenzoxazine der allgemeinen Formel III 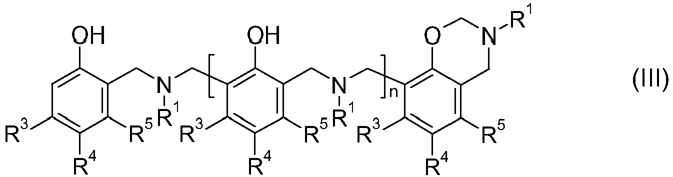 in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet,
wobei R⁶ ein Wasserstoffatom oder einen C₁- bis C₄-Alkylrest bezeichnet,
in der die Substituenten R², R³, R⁴ und R⁵ unabhängig voneinander für Wasserstoffatome oder Hydrocarbylreste mit jeweils 1 bis 3000 Kohlenstoffatomen, stehen, welche durch ein oder mehrere Heteroatome aus der Gruppe O und S und/oder durch eine oder mehrere Gruppierungen NR⁶ unterbrochen sein können, wobei R⁶ die oben genannte Bedeutung hat, und
in der n für eine ganze Zahl von 0 bis 18 steht,
mit der Maßgabe, dass mindestens einer der Substituenten R¹, R³, R⁴ oder R⁵ 13 bis 3000 Kohlenstoffatome aufweist und die übrigen Substituenten aus der Gruppe R¹, R³, R⁴ oder R⁵, wenn sie für Hydrocarbylreste stehen, jeweils 1 bis 20 Kohlenstoffatome aufweisen, offenbart.

Weiterhin werden zweikernige phenolische Verbindungen der allgemeinen Formel IV 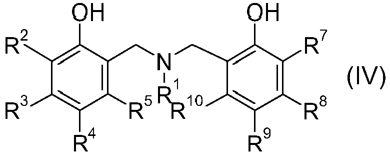 in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet,
wobei R⁶ ein Wasserstoffatom oder einen C₁- bis C₄-Alkylrest bezeichnet, und
in der die Substituenten R², R³, R⁴, R⁵, R⁷, R⁸, R⁹ und R¹⁰ unabhängig voneinander für Wasserstoffatome oder Hydrocarbylreste mit jeweils 1 bis 3000 Kohlenstoffatomen, stehen, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
mit der Maßgabe, dass mindestens einer der Substituenten R¹, R², R³, R⁴, R⁵, R⁷, R⁸, R⁹ und R¹⁰ 13 bis 3000 Kohlenstoffatome aufweist und die übrigen Substituenten aus der Gruppe R¹, R², R³, R⁴, R⁵, R⁷, R⁸, R⁹ und R¹⁰, wenn sie für Hydrocarbylreste stehen, jeweils 1 bis 20 Kohlenstoffatome aufweisen, offenbart.

Erfindungsgemäß werden phenolische Verbindungen der allgemeinen Formel V 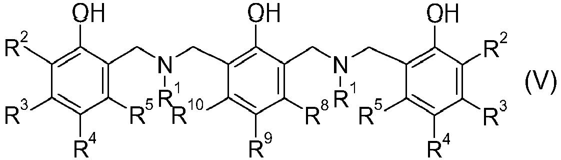 in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet,
wobei R⁶ ein Wasserstoffatom oder einen C₁- bis C₄-Alkylrest bezeichnet,
in der die Substituenten R², R³, R⁴, R⁵, R⁸, R⁹ und R¹⁰ unabhängig voneinander für Wasserstoffatome oder Hydrocarbylreste mit jeweils 1 bis 3000 Kohlenstoffatomen, stehen, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
wobei der Substituent R⁹ auch für einen Rest der Formel Z' stehen kann 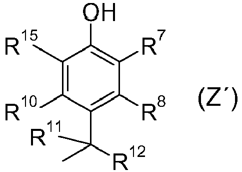 in dem die Substituenten R⁷, R⁸ und R¹⁰ die vorgenannten Bedeutungen haben, der Substituent R⁷ auch ein aus einem Tetrahydrobenzoxazin der allgemeinen Formel I abgeleiteter Rest sein kann, der Sustituent R¹⁵ für Wasserstoff oder einen aus einem Tetrahydrobenzoxazin der allgemeinen Formel I abgeleiteten Rest steht und die Substituenten R¹¹ und R¹² gleich oder verschieden sein können und Wasserstoff oder einen C₁- bis C₁₀-Alkylrest bezeichnen, und
mit der Maßgabe, dass mindestens einer der Substituenten R¹, R², R³, R⁴, R⁵, R⁸, R⁹ und R¹⁰ 13 bis 3000, vorzugsweise 26 bis 3000 Kohlenstoffatome aufweist und die übrigen Substituenten aus der Gruppe R¹, R², R³, R⁴, R⁵, R⁸, R⁹ und R¹⁰, wenn sie für Hydrocarbylreste stehen, jeweils 1 bis 20 Kohlenstoffatome aufweisen, verwendet bzw. eingesetzt.

Die Erfindung wir anhand der folgenden, nicht einschränkenden Beispiele näher erläutert.

### Herstellungsbeispiele

### Beispiel 1:

Verbindung der Formel Vb durch Umsetzung von 4-Polyisobutenylphenol mit 3-Methyl-6,8-di-tert.-butyltetrahydrobenzoxazin

In einem 500 ml-Vierhalskolben wurden 120 g 4-Polyisobutenylphenol (auf Basis des im Handel erhältlichen hochreaktiven Polyisobutens Glissopal® 1000 mit einem Mₙ von 1000) bei Raumtemperatur in 100 ml Toluol vorgelegt. 52 g 3-Methyl-6,8-di-tert.-butyltetrahydrobenzoxazin wurden rasch zugesetzt. Der Kolbeninhalt wurde bis zum Rückfluß erwärmt und 2 Stunden unter Rückfluß gerührt. Nach Abkühlung auf Raumtemperatur wurde der Kolbeninhalt mit Methanol gewaschen und die Toluolphase wurde bei 150°C und 5 mbar eingedampft. Man erhielt 113 g Produkt in Form eines hellen, klaren, zähen Öls.
1H-NMR (400 MHz, 16 Scans, CDCl₃):
Die Tetrahydrobenzoxazin-Signale (δ = 4,70 ppm, 2H, -CH₂-N(-CH₃)-CH₂-O-; δ = 3,82 ppm, 2H, -CH₂-N(-CH₃)-CH₂-O-;) sowie das Signal für ein ortho-Proton des 4-Polyisobutenylphenols (δ = 6,70 ppm) sind vollständig verschwunden;
Vorhanden sind im Bereich δ = 3,8-3,5 ppm die Benzylprotonen, im Bereich δ = 2,6-2,0 ppm die Protonen der N-Methylgruppe und im Bereich δ = 6,9-7,2 ppm die Aromatenprotonen.

### Beispiel 2:

Verbindung der Formel Ve durch Umsetzung von 4-Polyisobutenylphenol mit 3-(Dimethylamino)-propyl-6,8-di-tert.-butyltetrahydrobenzoxazin

In einem 1000 ml-Vierhalskolben wurden 170 g 4-Polyisobutenylphenol (auf Basis des im Handel erhältlichen hochreaktiven Polyisobutens Glissopal® 1000 mit einem Mₙ von 1000) bei Raumtemperatur in 100 ml Toluol vorgelegt. 100 g 3-(Dimethylamino)propyl-6,8-di-tert.-butyltetrahydrobenzoxazin wurden rasch zugesetzt. Der Kolbeninhalt wurde bis zum Rückfluß erwärmt und 2 Stunden unter Rückfluß gerührt. Nach Abkühlung auf Raumtemperatur wurde der Kolbeninhalt mit Methanol gewaschen und die Toluolphase wurde bei 90°C und 5 mbar eingedampft. Man erhielt 225 g Produkt in Form eines hellen, klaren, zähen Öls.
1H-NMR (400 MHz, 16 Scans, CDCl₃):
5-10% Rest-Tetrahydrobenzoxazin-Signale (δ = 4,70 ppm, 2H, -CH₂-N(-CH₃)-CH₂-O-; δ = 3,82 ppm, 2H, -CH₂-N(-CH₃)-CH₂-O-) sowie ein geringes Restsignal für ein ortho-Proton des 4-Polyisobutenylphenols (δ = 6,70 ppm) zeigen 90 bis 95 % Umsatz; Vorhanden sind im Bereich δ = 3,8-3,4 ppm die Benzylprotonen, im Bereich δ = 2,6-2,0 ppm die Protonen der N-Methyl- und N-Methylengruppen und im Bereich δ = 7,0-7,3 ppm die Aromatenprotonen.

### Beispiel 3:

Verbindung der Formel Vc durch Umsetzung von 4-Polyisobutenylphenol mit 3,6-Dimethyl-8-tert.-butyltetrahydrobenzoxazin

In einem 1000 ml-Vierhalskolben wurden 240 g 4-Polyisobutenylphenol (auf Basis des im Handel erhältlichen hochreaktiven Polyisobutens Glissopal® 1000 mit einem Mₙ von 1000) bei Raumtemperatur in 200 ml Toluol vorgelegt. 88 g 3,6-Dimethyl-8-tert.-butyl-tetrahydrobenzoxazin wurden rasch zugesetzt. Der Kolbeninhalt wurde bis zum Rückfluß erwärmt und 2 Stunden unter Rückfluß gerührt. Die Toluolphase wurde bei 150°C und 5 mbar eingedampft. Man erhielt 313 g Produkt in Form eines hellen, klaren, zähen Öls.
1H-NMR (400 MHz, 16 Scans, CDCl₃):
Rest-Tetrahydrobenzoxazin-Signale (δ = 4,70 ppm, 2H, -CH₂-N(-CH₃)-CH₂-O-; δ = 3,82 ppm, 2H, -CH₂-N(-CH₃)-CH₂-O-) sowie ein geringes Restsignal für ein ortho-Proton des 4-Polyisobutenylphenols (δ = 6,70 ppm) zeigen 80 % Umsatz;
Vorhanden sind im Bereich δ = 3,8-3,4 ppm die Benzylprotonen, im Bereich δ = 2,6-2,0 ppm die Protonen der N-Methylgruppe und im Bereich δ = 7,0-7,3 ppm die Aromatenprotonen.

### Beispiel 4 (nicht erfindungsgemäß):

Verbindung der Formel IIIv durch Umsetzung von 3-Methyl-6-polyisobutenyltetrahydrobenzoxazin mit 3-Methyl-6-tert.-butyltetrahydrobenzoxazin In einer 500 ml-Verdampferbirne wurden 100 g 3-Methyl-6-polyisobutenyltetrahydrobenzoxazin (auf Basis des im Handel erhältlichen hochreaktiven Polyisobutens Glissopal® 1000 mit einem Mₙ von 1000) bei Raumtemperatur in 200 ml Toluol vorgelegt. 16,4 g 3-Methyl-6-tert.-butyltetrahydrobenzoxazin wurden rasch zugesetzt. Das Toluol wurde am Rotationsverdampfer bei 100°C / 5 mbar abdestilliert und es wurde 4 Stunden bei 160°C / 900 mbar gerührt. Man erhielt 110 g Produkt in Form eines viskosen, zähen Öls.
1H-NMR (400 MHz, 16 Scans, CDCl₃):
Bezogen auf das Integral der Methylprotonen (Benzoxazin: 2,45-2,65 ppm; 2-Hydroxybenzylamin: 2,05-2,30 ppm) sind zwei Phenoleinheiten mit einem Benzoxazin verknüpft.

### Beispiel 5 (nicht erfindungsgemäß):

Verbindung der Formel IIIw durch Umsetzung von 3-Methyl-6-polyisobutenyltetrahydrobenzoxazin mit 3-Methyl-6-tert.-butyltetrahydrobenzoxazin

In einer 500 ml-Verdampferbirne wurden 50 g 3-Methyl-6-polyisobutenyltetrahydrobenzoxazin (auf Basis des im Handel erhältlichen hochreaktiven Polyisobutens Glissopal® 1000 mit einem Mₙ von 1000) bei Raumtemperatur in 200 ml Toluol vorgelegt. 41 g 3-Methyl-6-tert.-butyltetrahydrobenzoxazin wurden rasch zugesetzt. Das Toluol wurde am Rotationsverdampfer bei 100°C / 5 mbar abdestilliert und es wurde 4 Stunden bei 160°C / 900 mbar gerührt. Man erhielt 75 g Produkt in Form eines viskosen, zähen Öls.
1H-NMR (400 MHz, 16 Scans, CDCl₃):
Bezogen auf das Integral der Methylprotonen, (Benzoxazin: 2,45-2,65ppm; 2-Hydroxybenzylamin: 2,05-2,30 ppm) sind 9 Phenoleinheiten mit einem Benzoxazin verknüpft.

### Beispiel 6:

Verbindung der Formel VIc durch Umsetzung von 3-Methyl-6-polyisobute-nyltetrahydrobenzoxazin und 3-Methyl-6,8-di-tert.-butyltetrahydrobenzoxazin mit Bisphenol A

In einem 1000 ml-Vierhalskolben wurden 130 g 3-Methyl-6-polyisobutenyltetrahydrobenzoxazin (auf Basis des im Handel erhältlichen hochreaktiven Polyisobutens Glissopal® 1000 mit einem Mₙ von 1000) 78,5 g 3-Methyl-6,8-di-tert.-butyltetrahydrobenzoxazin, 22,8 g Bisphenol A und 200 ml Toluol 2 Stunden unter Rückfluß gerührt. Der Kolbeninhalt wurde bei 140°C und 5 mbar eingedampft. Man erhielt 194,5 g Produkt in Form eines hellen, klaren, zähen Öls.
1H-NMR (400 MHz, 16 Scans, CDCl₃):
5% Rest-Tetrahydrobenzoxazin-Signale (δ = 4,70 ppm, 2H, -CH₂-N(-CH₃)-CH₂-O-; δ = 3,82 ppm, 2H, -CH₂-N(-CH₃)-CH₂-O-) sowie ein geringes Restsignal für ein ortho-Proton des Bisphenol A (δ = 6,60 ppm) zeigen 95 % Umsatz;
Vorhanden sind im Bereich δ = 3,8-3,4 ppm die Benzylprotonen, im Bereich δ = 2,6-2,0 ppm die Protonen der N-Methylgruppen und im Bereich δ = 6,7-7,3 ppm die Aromatenprotonen.

### Beispiel 7: Verbindung der Formel Vn durch Umsetzung von 4-Polyisobutenylphenol mit 3-(Dimethylamino)-propyl-6-methyl-8-tert.-butyltetrahydrobenzoxazin

In einem 1000 ml-Vierhalskolben wurden 300 g 4-Polyisobutenylphenol (auf Basis des im Handel erhältlichen hochreaktiven Polyisobutens Glissopal® 1000 mit einem Mₙ von 1000) bei Raumtemperatur in 190 g Solvesso® 150 (Solvent Naphta Heavy) vorgelegt. 145 g 3-(Dimethylamino)-propyl-6-methyl-8-tert.-butyltetrahydrobenzoxazin wurden rasch zugesetzt. Der Kolbeninhalt wurde 4 Stunden bei 150°C gerührt. Man erhielt 630 g einer hellen, klaren Lösung des Produktes (70 Gew.-% Feststoffgehalt). Für die analytische Bestimmung wurde ein Probe bei 150°C und 3 mbar eingedampft.
1H-NMR (400 MHz, 16 Scans, CDCl₃):
Rest-Tetrahydrobenzoxazin-Signale (δ = 4,80 ppm, 2H, -CH₂-N(-CH₃)-CH₂-O-; δ = 3,91 ppm, 2H, -CH₂-N(-CH₃)-CH₂-O-) sowie ein geringes Restsignal für ein ortho-Proton des 4-Polyisobutenylphenols (δ = 6,70 ppm) zeigen 90 % Umsatz;
Vorhanden sind im Bereich δ = 3,8-3,4 ppm die Benzylprotonen, im Bereich δ = 2,6-2,0 ppm die Protonen der N-Methylgruppe und im Bereich δ = 6,7-7,3 ppm die Aromatenprotonen.

### Anwendungsbeispiele

### Beispiel 8: Überprüfung der thermischen Stabilität von Turbinenkraftstoff (jet fuel)

Es wurde ein Turbinenkraftstoff der Spezifikation Jet A-1 nach DEF STAN 91-91 bzw. ASTM D 1655 eingesetzt. Die Additivierung erfolgte mit jeweils 100 mg/l der Verbindungen aus den Herstellungsbeispielen 1 bis 6.

In einem Dreihals-Glaskolben, der mit Rührer, Rückflusskühler und Thermometer versehen war, wurden zunächst bei Raumtemperatur 5 I Luft innerhalb 1 h durch 150 ml des zu untersuchenden Kraftstoffs geleitet. Anschließend wurde der Kraftstoff mit einem Ölbad auf 140 °C erhitzt und weitere 5 h bei dieser Temperatur gerührt. Nach Abkühlen auf Raumtemperatur wurde die gesamte Kraftstoffmenge über einen 0,45 µm Membranfilter filtriert. Anschließend wurde der Filterrückstand nach 45 min Trocknen im Trockenschrank bei 115 °C und anschließender 2-stündiger Trocknung unter Vakuum im Exsiccator gravimetrisch bestimmt:
Blindwert (ohne Additiv): 8,9 mg
erfindungsgemäß additiviert mit jeweils 100 mg/l der folgenden Verbindungen:
Verbindung der Formel Vb (Herstellungsbeispiel 1): 1,8 mg
Verbindung der Formel Ve (Herstellungsbeispiel 2): 1,0 mg
Verbindung der Formel Vc (Herstellungsbeispiel 3): 1,0 mg
Verbindung der Formel IIIv (Herstellungsbeispiel 4): 1,6 mg
Verbindung der Formel IIIw (Herstellungsbeispiel 5): 2,5 mg
Verbindung der Formel VIc (Herstellungsbeispiel 6): 1,0 mg

Durch den Einsatz des erfindungsgemäß verwendeten Additivs konnte die durch thermische Belastung des Turbinenkraftstoffs entstehende Partikelmenge deutlich reduziert werden.

### Beispiel 9: Verbesserung der thermischen Stabilität von Turbinenkraftstoff (jet fuel)

Es wurde ein Turbinenkraftstoff in Anlehnung an die Spezifikation Jet A-1 nach DEF STAN 91-91 bzw. ASTM D 1655 eingesetzt. Die Überprüfung der Thermostabilität erfolgte nach der JFTOT-Breakpoint-Methode nach ASTM D 3241. Bei dem nicht additivierten Turbinenkraftstoff wurde ein Wert von 250°C ermittelt. Mit Kraftstoffen, die mit jeweils 100 mg/l eines nachfolgend aufgeführten erfindungsgemäß verwendeten Additivs additiviert waren, wurden die folgenden Werte gemessen:
Verbindung der Formel Vb (Herstellungsbeispiel 1): 280°C
Verbindung der Formel Ve (Herstellungsbeispiel 2): 260°C
Verbindung der Formel Vc (Herstellungsbeispiel 3): 280°C

### Beispiel 10: Überprüfung der Wasserverträglichkeit von Turbinenkraftstoff

Es wurden die Verbindung der Formel Vb aus Herstellungsbeispiel 1 und der gleiche Turbinenkraftstoff wie in Beispiel 8 und 9 verwendet.

Gemäß DIN 51415 bzw. ASTM D 1094 wurde nach Zugabe von 100 mg/l der Verbindung der Formel Vb die Wasserverträglichkeit des Turbinenkraftstoffes und damit die unerwünschte Neigung, Emulsionen zu bilden, bestimmt. Hierzu wurden 80 ml des additivierten Turbinenkraftstoffes und 20 ml Wasser in definierter Weise intensiv geschüttelt. Danach erfolgte eine visuelle Bewertung der Phasentrennschichten nach jeweils 1, 5, 30 und 60 Minuten. Bereits 5 Minuten nach Wasserzugabe erhielt man eine vollständige Trennung von Kraftstoff und Wasser, es blieben keine Emulsionsanteile zurück.

### Beispiel 11: Überprüfung der Wasserabtrennungseigenschaften von Turbinenkraftstoff

Es wurde ein Turbinenkraftstoff der Spezifikation Jet A-1 nach DEF STAN 91-91 bzw. ASTM D 1655 eingesetzt. Die Überprüfung der Neigung von Turbinenkraftstoffen bezüglich ihrer Wasserabtrennungseigenschaften erfolgte nach ASTM D 3948 ("MSEP"-Test). Charakteristisch für diese Messungen ist die Verwendung eines Standard-Koaleszierfilters mit abschließender Trübungsmessung der Kraftstoffphase. Bei der Messung wurden die erfindungsgemäß verwendeten Additive in Kombination mit dem Antioxidans 2,6-Di-tert.-butyl-4-methylphenol ("BHT") und dem Metalldeaktivator N,N'-Disalicyliden-1,2-diaminopropan in einem hierfür üblichen Lösungsmittel geprüft. Die Dosierung der erfindungsgemäß verwendeten Additive betrug jeweils 215 mg/l (bezogen auf deren 100% igen Wirkstoffgehalt). Es wurden folgende Benotungen für das Trübungsverhalten ermittelt [relative Bewertungsskala von 0 (schlechteste Note) bis 100 (beste Note)] :
Blindwert (ohne Additiv): 98
Verbindung der Formel Vb (Herstellungsbeispiel 1): 97
Verbindung der Formel Vc (Herstellungsbeispiel 3): 98

Es traten keine Verschlechterungen im Vergleich mit nicht-additiviertem Turbinenkraftstoff auf.

## Patentansprüche

1. Verwendung von phenolischen Verbindungen der allgemeinen Formel V 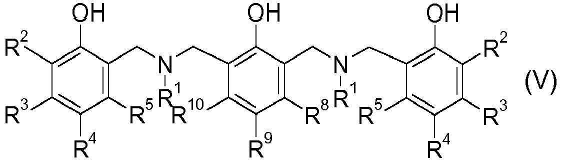 in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet,
wobei R⁶ ein Wasserstoffatom oder einen C₁- bis C₄-Alkylrest bezeichnet,
in der die Substituenten R², R³, R⁴, R⁵, R⁸, R⁹ und R¹⁰ unabhängig voneinander für Wasserstoffatome oder Hydrocarbylreste mit jeweils 1 bis 3000 Kohlenstoffatomen, stehen, welche durch ein oder mehrere Heteroatome aus der Gruppe O und S und/oder durch eine oder mehrere Gruppierungen NR⁶ unterbrochen sein können, wobei R⁶ die oben genannte Bedeutung hat,
wobei der Substituent R⁹ auch für einen Rest der Formel Z' stehen kann 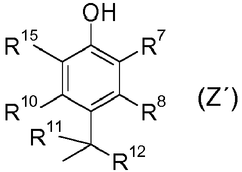 in dem die Substituenten R⁷, R⁸ und R¹⁰ unabhängig voneinander für Wasserstoffatome, Hydroxylgruppen oder Hydrocarbylreste mit jeweils 1 bis 3000 Kohlenstoffatomen stehen, welche durch ein oder mehrere Heteroatome aus der Gruppe O und S und/oder durch eine oder mehrere Gruppierungen NR⁶ unterbrochen sein können, wobei R⁶ die oben genannte Bedeutung hat, der Substituent R⁷ auch ein aus einem Tetrahydrobenzoxazin der allgemeinen Formel I 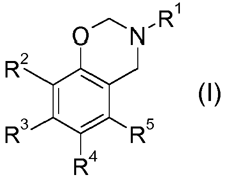 abgeleiteter Rest sein kann, der Substituent R¹⁵ für Wasserstoff oder einen aus einem Tetrahydrobenzoxazin der allgemeinen Formel I abgeleiteten Rest steht und die Substituenten R¹¹ und R¹² gleich oder verschieden sein können und Wasserstoff oder einen C₁- bis C₁₀-Alkylrest bezeichnen, und
mit der Maßgabe, dass mindestens einer der Substituenten R¹, R², R³, R⁴, R⁵, R⁸, R⁹ und R¹⁰ 13 bis 3000 Kohlenstoffatome aufweist und die übrigen Substituenten aus der Gruppe R¹, R², R³, R⁴, R⁵, R⁸, R⁹ und R¹⁰, wenn sie für Hydrocarbylreste stehen, jeweils 1 bis 20 Kohlenstoffatome aufweisen,
als Antioxidantien zur Stabilisierung von unbelebtem organischen Material gegen die Einwirkung von Licht, Sauerstoff und Wärme.

2. Verwendung von phenolischen Verbindungen nach Anspruch 1 als Antioxidantien in Turbinenkraftstoffen.

3. Verwendung von phenolischen Verbindungen nach Anspruch 2 als Antioxidantien zur Verbesserung der thermischen Stabilität von Turbinenkraftstoffen.

4. Turbinenkraftstoffzusammensetzung, enthaltend einen Turbinenkraftstoff und wenigstens eine phenolische Verbindung, wie in Anspruch 1 definiert.

5. Verwendung von phenolischen Verbindungen nach Anspruch 1 als Stabilisatoren zur Verbesserung der thermischen Stabilität von Ottokraftstoffen und Mitteldestillat-Kraftstoffen.

6. Verwendung von phenolischen Verbindungen nach Anspruch 1 als Stabilisatoren in Ottokraftstoffen und Mitteldestillat-Kraftstoffen zur Verhinderung von Ablagerungen im Kraftstoffsystem und/oder Verbrennungssystem von Otto- bzw. Dieselmotoren.

7. Otto- oder Dieselkraftstoff, enthaltend neben mindestens einer phenolischen Verbindungen, wie in Anspruch 1 definiert, als weiteres Kraftstoffadditiv mindestens ein Detergens.

## Claims

1. The use of phenolic compounds of the general formula V 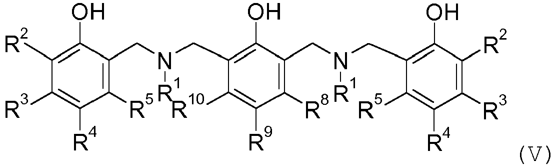 in which the substituent R¹ is a hydrocarbyl radical which has from 1 to 3000 carbon atoms and may be interrupted by one or more heteroatoms from the group of O and S and/or may be interrupted by one or more NR⁶ moieties,
where R⁶ is a hydrogen atom or a C₁- to C₄-alkyl radical,
in which the substituents R², R³, R⁴, R⁵, R⁸, R⁹ and R¹⁰ are each independently hydrogen atoms or hydrocarbyl radicals which have in each case from 1 to 3000 carbon atoms and may be interrupted by one or more heteroatoms from the group of O and S and/or by one or more NR⁶ moieties, where R⁶ is as defined above,
where the substituent R⁹ may also be a radical of the formula Z' 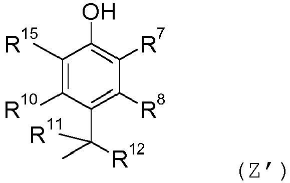 in which the substituents R⁷, R⁸ and R¹⁰ are each independently hydrogen atoms, hydroxyl groups or hydrocarbyl radicals which have in each case from 1 to 3000
carbon atoms and may be interrupted by one or more heteroatoms from the group of O and S and/or by one or more NR⁶ moieties, where R⁶ is as defined above, the substituent
R⁷ may also be a radical derived from a tetrahydrobenzoxazine of the general formula I 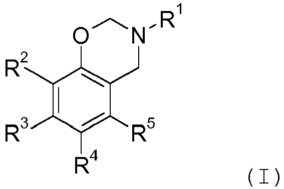 the substituent R¹⁵ is hydrogen or a radical derived from a tetrahydrobenzoxazine of the general formula I, and the substituents R¹¹ and R¹² may be the same or different and are each hydrogen or a C₁- to C₁₀-alkyl radical, and
with the proviso that at least one of the substituents R¹, R², R³, R⁴, R⁵, R⁸, R⁹ and R¹⁰ has from 13 to 3000 carbon atoms, and the remaining substituents from the group of R¹, R², R³, R⁴, R⁵, R⁸, R⁹ and R¹⁰, when they are hydrocarbyl radicals, each have from 1 to 20 carbon atoms, as antioxidants for stabilizing inanimate organic material against the action of light, oxygen and heat.

2. The use of phenolic compounds according to claim 1 as antioxidants in turbine fuels.

3. The use of phenolic compounds according to claim 2 as antioxidants for improving the thermal stability of turbine fuels.

4. A turbine fuel composition comprising a turbine fuel and at least one phenolic compound as defined in claim 1.

5. The use of phenolic compounds according to claim 1 as stabilizers for improving the thermal stability of gasoline fuels and middle-distillate fuels.

6. The use of phenolic compounds according to claim 1 as stabilizers in gasoline fuels and middle-distillate fuels for preventing deposits in the fuel system and/or combustion system of gasoline and/or diesel engines.

7. A gasoline or diesel fuel comprising at least one of phenolic compounds as defined in claim 1 and as a further fuel additive at least one detergent.

## Revendications

1. Utilisation de composés phénoliques de la formule générale V : 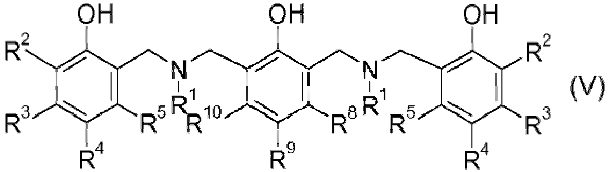 dans laquelle le substituant R¹ désigne un radical hydrocarbyle de 1 à 3 000 atomes de carbone, qui peut être interrompu par un ou plusieurs hétéroatomes du groupe constitué par O et S et/ou par un ou plusieurs groupes NR⁶,
R⁶ désignant un atome d'hydrogène ou un radical alkyle en C₁ à C₄,
dans laquelle les substituants R², R³, R⁴, R⁵, R⁸, R⁹ et R¹⁰ représentent indépendamment les uns des autres des atomes d'hydrogène ou des radicaux hydrocarbyle contenant chacun 1 à 3 000 atomes de carbone, qui peuvent être interrompus par un ou plusieurs hétéroatomes du groupe constitué par O et S et/ou par un ou plusieurs groupes NR⁶, R⁶ ayant la signification indiquée précédemment,
le substituant R⁹ pouvant également représenter un radical de la formule Z' : 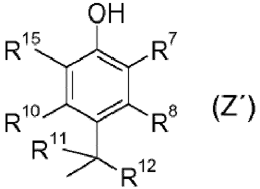 dans laquelle les substituants R⁷, R⁸ et R¹⁰ représentent indépendamment les uns des autres des atomes d'hydrogène, des groupes hydroxyle ou des radicaux hydrocarbyle contenant chacun 1 à 3 000 atomes de carbone, qui peuvent être interrompus par un ou plusieurs hétéroatomes du groupe constitué par O et S et/ou par un ou plusieurs groupes NR⁶, R⁶ ayant la signification indiquée précédemment, le substituant R⁷ pouvant également être un radical dérivé d'une tétrahydrobenzoxazine de la formule générale I : 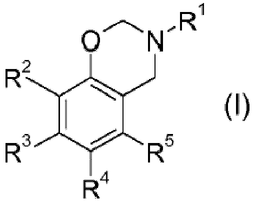 le substituant R¹⁵ représentant l'hydrogène ou un radical dérivé d'une tétrahydrobenzoxazine de la formule générale I, et les substituants R¹¹ et R¹² pouvant être identiques ou différents, et désignant l'hydrogène ou un radical alkyle en C₁ à C₁₀,
à condition qu'au moins un des substituants R¹, R², R³, R⁴, R⁵, R⁸, R⁹ et R¹⁰ comprenne 13 à 3 000 atomes de carbone, et les autres substituants du groupe constitué par R¹, R², R³, R⁴, R⁵, R⁸, R⁹ et R¹⁰ comprennent chacun 1 à 20 atomes de carbone lorsqu'ils représentent des radicaux hydrocarbyle,
en tant qu'antioxydants pour la stabilisation d'un matériau organique non vivant contre l'effet de la lumière, de l'oxygène et de la chaleur.

2. Utilisation de composés phénoliques selon la revendication 1 en tant qu'antioxydants dans des carburants de turbines.

3. Utilisation de composés phénoliques selon la revendication 2 en tant qu'antioxydants pour améliorer la stabilité thermique de carburants de turbines.

4. Composition de carburant de turbine, contenant un carburant de turbine et au moins un composé phénolique tel que défini dans la revendication 1.

5. Utilisation de composés phénoliques selon la revendication 1 en tant que stabilisateurs pour améliorer la stabilité thermique d'essences et de carburants à base de distillats moyens.

6. Utilisation de composés phénoliques selon la revendication 1 en tant que stabilisateurs dans des essences et des carburants à base de distillats moyens pour inhiber les dépôts dans le système de carburant et/ou le système de combustion de moteurs à essence ou diesel.

7. Essence ou carburant diesel, contenant, outre au moins un composé phénolique tel que défini dans la revendication 1, au moins un détergent en tant qu'additif de carburant supplémentaire.
